# EUROPEAN PATENT APPLICATION

(11) **EP 2 984 941 A1**
(43) Date of publication of application: **17.02.2016**
(21) Application number: 15179168.8
(22) Date of filing: 22.08.2011
(51) Int. Cl.: A23L 33/00, A23L 33/10, A23L 33/17, A61K 38/01, A61K 36/48

(54) **NUTRITIONAL PRODUCTS HAVING IMPROVED ORGANOLEPTIC PROPERTIES**

(30) Priority: 24.08.2010 US 376503 P
(62) Divisional of application: 11760896.8
(71) Applicant: Abbott Laboratories, Abbott Park, IL 60064 (US)
(72) Inventor: CORDLE, Christopher T., Centerburg, OH Ohio 43011-0016 (US); LUEBBERS, Steven T., Columbus, OH Ohio 43235 (US); WILLIAMS, Larry W., New Albany, OH Ohio 43054 (US); BAXTER, Jeffrey Harris, Westerville, OH Ohio 43081 (US); DUSKA-MCEWEN, Geralyn, Gahann, OH Ohio 43230 (US)
(74) Representative: Boult Wade Tennant

(57) **Abstract**

Disclosed are nutritional products comprising pea protein hydrolysates as a primary or sole source of protein. The nutritional products include nutritional liquids, powders, bars, and other food products, wherein the resulting nutritional products are physically stable over shelf life and provide favorable organoleptic properties. These nutritional products may be used as a nutritional source for individuals who are intolerant and/or allergic to cow milk proteins and soy proteins and/or who have malabsorption, maldigestion, or other gastrointestinal conditions.

## Description

### FIELD OF THE DISCLOSURE

The present disclosure relates to nutritional products comprising pea protein hydrolysates. More particularly, the present disclosure relates to infant, pediatric and adult nutritional products comprising pea protein hydrolysates having lowered immunological reactivity and improved flavor and organoleptic properties, including emulsion stability.

### BACKGROUND OF THE DISCLOSURE

Adult, pediatric, and infant manufactured nutritional liquids and powders comprising a targeted selection of nutritional ingredients are well known and widely available, some of which may provide a sole source of nutrition, while others may provide a supplemental source. These nutritionals include powders that can be reconstituted with water or other aqueous liquid, as well as concentrated and ready-to-drink nutritional liquids such as milk or protein based emulsions or non-emulsified or substantially clear liquids. These nutritional liquids arc especially useful when formulated with selected nutritional ingredients.

Many individuals, and particularly infants, including preterm infants, experience intolerance to certain nutritional ingredients in the liquids and powders (formula intolerance). The terms "intolerance" and "formula intolerance" are used interchangeably herein. Intolerance is a non-immune system associated reaction and may be evidenced by behavior or stool or feeding pattern changes such as increased vomiting, an increased number of stools, or more watery stools as compared to individuals who tolerate nutritional ingredients well. Intolerance is most often indicated by gastrointestinal symptoms (e.g. emesis, stool patterns and gas) as well as behavioral characteristics (e.g. acceptance of formula, fussing and crying).

Intolerance can be contrasted with the allergic-type reactions that some individuals, and especially infants, exhibit to certain nutritional compositions. These allergic-type reactions are immune system associated, and may be caused by the individual's sensitivity to the protein present (generally cow milk protein) in the nutritional. These immune system associated allergies or sensitivities often result in cutaneous, respiratory or gastrointestinal symptoms such as vomiting and diarrhea.

Many individuals who exhibit allergies or sensitivities to cow milk proteins are able to utilize nutritional products based on soy proteins, and in particular, isolated soy proteins.

Hydrolysate-based formulas, some of which may be hypoallergenic, contain protein that has been hydrolyzed or broken down into short peptide fragments and amino acids and as a result is immunologically substantially inactive. Individuals who exhibit reactions to cow milk and soy proteins often will not react to hydrolysate formulas because their immune system does not recognize the hydrolyzed protein as the intact protein that causes their symptoms. Individuals who exhibit immune system-associated reactions to nutritional compositions including cow milk and/or soy proteins may also exhibit non-immune system associated reactions (formula intolerance), as previously described.

Although products based on extensively hydrolyzed proteins or amino acid mixtures are generally acceptable for individuals who are allergic to cow milk and soy proteins, they suffer from significant taste and organoleptic issues. While many attempts to avoid formula allergies and intolerance have been made by preparing nutritional compositions using hydrolyzed proteins, and particularly hydrolyzed soy proteins, it was not previously known how to formulate a nutritional product using hydrolyzed proteins without significantly adversely affecting the organoleptic properties of the product, and in particular, taste. For example, the use of hydrolyzed proteins commonly results in nutritional products that easily separate and/or precipitate out of solution, and further, are bitter, salty, sour, and/or beany in flavor.

The present disclosure is therefore directed to nutritional products in the form of nutritional liquids, powders, bars, and other food products comprising pea protein hydrolysates as the primary or sole source of protein. The pea protein hydrolysate has a degree of hydrolysis such that the resulting nutritional products are physically stable over shelf life and provide favorable organoleptic properties, while remaining substantially immunologically inactive. The nutritional product may also be used as a nutritional source for individuals who are intolerant and/or allergic to proteins other than pea proteins and/or who have malabsorption, maldigestion, or other gastroinstestinal conditions.

### SUMMARY OF THE DISCLOSURE

One embodiment is a nutritional product including a pea protein hydrolysate having an adjusted degree of hydrolysis of between about 7.0% to less than 19.0%, including between about 8.0% and about 16.0%, including between about 8% and about 13.5%, and also including between about 12.0% and 16.0%. Additionally, in one embodiment, less than 5% of the proteins in the pea protein hydrolysate have a molecular weight of greater than 3 kDa. The product is in a form selected from the group consisting of a liquid beverage (e.g., nutritional emulsions, substantially clear nutritional liquids, and gels), a powder, a food product, a nutritional bar, and a nutritional supplement. Desirably, the product is an infant formula.

Another embodiment is a nutritional product including a pea protein hydrolysate having less than 15,000 µg of immunologically active pea antigen per 1 gram of protein.

Another embodiment is a method of making a nutritional product comprising combining a protein, a fat, and a carbohydrate. The nutritional product includes a pea protein hydrolysate having an adjusted degree of hydrolysis of between about 7.0% and less than 19.0% as its primary or sole source of protein. In one embodiment, less than 5% of the proteins in the pea protein hydrolysate have a molecular weight of greater than 3 kDa. In another embodiment, the pea protein hydrolysate has less than 15,000 µg of immunologically active pea antigen per 1 gram of protein.

The nutritional products as described herein comprise pea protein hydrolysates as sole or primary source of protein that not only provide a nutritional source for individuals who are intolerant and/or allergic to cow milk proteins and/or soy proteins, but also is physically stable and/or has organoleptic benefits in each of the selected product forms. In many embodiments, described herein, pea protein hydrolysate is the sole protein source such that the nutritional product is hypoallergenic and suitable for use in food-allergic patients.

It has now been found that these intolerance and/or allergic issues can be minimized or eliminated by formulating the nutritional product with a pea protein hydrolysate having an adjusted degree of hydrolysis of between about 7.0% and less than 19.0%, which produces a specific molecular weight profile and antigen content. Specifically, the use of these pea protein hydrolysates has been found to have reduced immunological activity. Further, pea protein hydrolysates having an adjusted degree of hydrolysis of between about 7.0% and less than 19.0% provide improved flavor and other improved organoleptic properties as compared to nutritional products including other hydrolyzed vegetable protein sources. As such, utilizing the hydrolyzed pea protein of the present disclosure it is now possible to produce pleasant tasting, hypoallergenic, tolerant hydrolysate-based nutritionals.

### BRIEF DESCRIPTION OF THE DRAWINGS

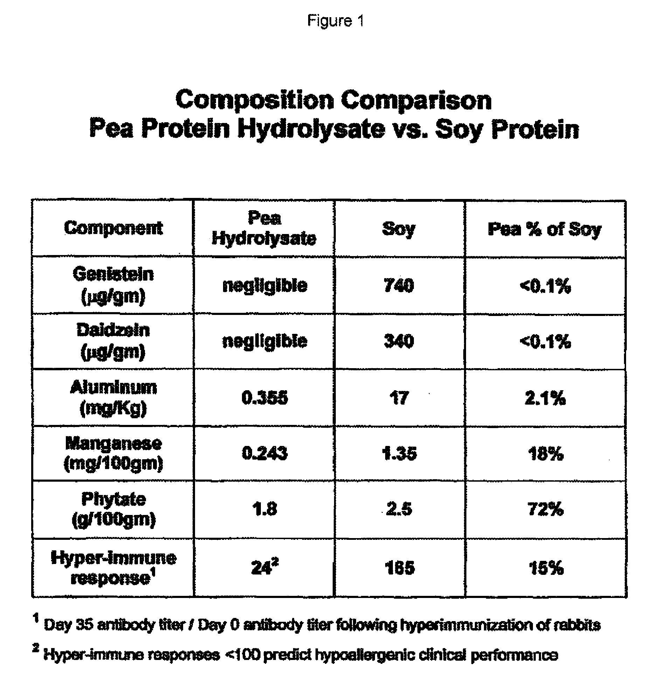

FIG. 1 is a chart depicting the composition comparison between pea protein hydrolysates of the present disclosure and intact soy protein.

### DETAILED DESCRIPTION OF THE DISCLOSURE

The nutritional products described herein comprise pea protein hydrolysates as a source of protein. In many embodiments, the nutritional products comprise pea protein hydrolysates as a sole protein source such that the nutritional product is hypoallergenic and suitable for use in food-allergic patients. These and other essential features of the nutritional products, as well as some of the many optional variations and additions, are described in detail hereafter.

The term "hypoallergenic" as used herein, unless otherwise specified, refers to a nutritional product that causes symptoms of an allergic reaction to food in less than 10% of food-allergic individuals.

The term "substantially clear nutritional liquid" as used herein, unless otherwise specified, refers to a non-emulsified or similar other liquid having a visibly clear or translucent appearance, which liquid may and typically will have a thin or watery texture with a consistency similar to that of a clear juice and most typically having a viscosity of less than about 25 centipoise as determined by a Brookfield viscometer at 22°C using a #1 spindle at 60 rpm.

The term "spray dried nutritional powder" as used herein, unless otherwise specified, refers to a nutritional powder wherein the majority of the components, including the pea protein hydrolysate, have been homogenized and subsequently subjected to a spray drying process during manufacturing. Additional ingredients can be added to the spray dried powder through dry blending so long as at least the pea protein hydrolysate has been previously homogenized and spray dried.

The term "adjusted degree of hydrolysis" as used herein, unless otherwise specified, refers to the degree of hydrolysis of a specified protein material, generally pea protein material, that has been adjusted or corrected to account for the measurable amino nitrogen content of the intact protein material. For example, if the intact, unmodified protein material has an amino nitrogen content of 8.68 and a protein that has been subjected to a hydrolysis procedure has a degree of hydrolysis of 16.68, the hydrolyzed protein has an adjusted degree of hydrolysis of 8.00.

The terms "fat" and "oil" as used herein, unless otherwise specified, are used interchangeably to refer to lipid materials derived or processed from plants or animals. These terms also include synthetic lipid materials so long as such synthetic materials are suitable for oral administration to humans.

The term "human milk oligosaccharide" or "HMO" refers generally to a number of complex carbohydrates found in human milk. Monomers found in milk oligosaccharides typically include D-glucose (Glc), D-galactose (Gal), N-acetylglucosamine (GlcNAC), L-fucose (Fuc), and sialic acid [N-acetylneuraminic acid (NeuAc)]. Elongation of these monomers may be achieved by attachment of GlcNAc residues linked in β1-3 or β1-4 linkage to a Gal residue followed by further addition of Gal in a β-1-3 or β-1-4 bond. Most HMOs carry lactose at their reducing end. From these monomers, a large number of core structures may be formed. Further variations may occur due to the attachment of lactosamine, Fuc, and/or NeuAc.

The term "shelf stable" as used herein, unless otherwise specified, refers to a nutritional product that remains commercially stable after being packaged and then stored at 18-24°C for at least 3 months, including from about 6 months to about 24 months, and also including from about 12 months to about 18 months.

The terms "nutritional composition" or "nutritional product" as used herein, are used interchangeably and, unless otherwise specified, refer to nutritional liquids, nutritional powders, nutritional bars, nutritional supplements, and any other nutritional food product as known in the art. The nutritional powders may be reconstituted to form a nutritional liquid, all of which comprise one or more of fat, protein and carbohydrate and are suitable for oral consumption by a human.

The term "nutritional liquid" as used herein, unless otherwise specified, refers to nutritional products in ready-to-drink liquid form, concentrated form, and nutritional liquids made by reconstituting the nutritional powders described herein prior to use.

The term "infant" as used herein, unless otherwise specified, refers to a child 12 months or younger. The term "toddler" as used herein, refers to a child from 6 months to three years of age. The term "child" as used herein, refers to a user from birth up to 13 years of age. The term "preterm infant" as used herein, refers to an infant born prior to 36 weeks of gestation.

All percentages, parts and ratios as used herein, are by weight of the total composition, unless otherwise specified. All such weights, as they pertain to listed ingredients, are based on the active level and, therefore, do not include solvents or by-products that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional products of the present disclosure may also be substantially free of any optional or selected essential ingredient or feature described herein, provided that the remaining nutritional product still contains all of the required ingredients or features as described herein. In this context, and unless otherwise specified, the term "substantially free" means that the selected nutritional product contains less than a functional amount of the optional ingredient, typically less than about 1%, including less than about 0.5%, including less than about 0.1%, and also including zero percent, by weight of such optional or selected essential ingredient.

The nutritional products may comprise, consist of, or consist essentially of the essential elements of the products as described herein, as well as any additional or optional element described herein or otherwise useful in nutritional product applications.

### Product Form

The nutritional compositions of the present disclosure including the pea protein hydrolysate may be formulated and administered in any known or otherwise suitable oral product form. Any solid, liquid, or powder form, including combinations or variations thereof, are suitable for use herein, provided that such forms allow for safe and effective oral delivery to the individual of the essential ingredients as also defined herein.

The nutritional compositions of the present disclosure include pea protein hydrolysates as described herein. The compositions may include pea protein hydrolysates as the sole source of protein (i.e., pea protein hydrolysates are 100% of the protein component of the composition) or they may include pea protein hydrolysates in combination with other hydrolyzed, partially hydrolyzed, or unhydrolyzed (intact) proteins as described herein. The compositions include any product form comprising the essential ingredients described herein, and which is safe and effective for oral administration. The nutritional compositions may be formulated to include only the essential ingredients described herein, or may be modified with optional ingredients to form a number of different product forms. The nutritional compositions of the present disclosure are preferably formulated as dietary product forms, which are defined herein as those embodiments comprising the essential ingredients of the present disclosure in a product form that then contains at least one of fat, protein, and carbohydrate, and preferably also contains vitamins, minerals, or combinations thereof. In many embodiments, the composition will comprise pea protein hydrolysate as the sole source of protein in combination with fat, carbohydrate, vitamins, and minerals to produce a hypoallergenic composition. One specific example is a hypoallergenic infant formula including 100% pea protein hydrolysates as the protein source, fat, carbohydrate, vitamins and minerals.

The nutritional compositions of the present disclosure may therefore include a variety of different product forms, including most any conventional or otherwise known food product form, some non-limiting examples of which include confectionary products, cereals, food condiments (e.g., spreads, powders, sauces, jams, jelly, coffee creamer or sweetener), pasta, baking or cooking materials (e.g., flour, fats or oils, butter or margarine, breading or baking mixes), salted or seasoned snacks (e.g., extruded, baked, fried), beverages (e.g., coffee, juice, carbonated beverage, non-carbonated beverage, tea), snack or meal replacement bars (e.g., Slimfast™ bars, Ensure™ bars, Zone perfect™ bars, Glucerna™ bars), smoothies, breakfast cereals, cheeses, gummie products, salted or unsalted crisp snacks (e.g., chips, crackers, pretzels), dips, baked goods (e.g., cookies, cakes, pies, pastries, bread, bagels, croutons, dressings, dry mixes (e.g., mixes for muffins, cookies, waffles, pancakes, beverages), frozen desserts (e.g., ice cream, popsicles, fudge bars, crushed ice, frozen yogurt), pasta, processed meats (e.g., corn dogs, hamburgers, hotdogs, sausage, pepperoni), pizza, pudding, flavored or unflavored gelatin, refrigerated dough (e.g., cookies, bread, brownies), yogurt or yogurt-based drinks, frozen yogurt, soups, vegetable-based burgers, and popcorn-based snacks.

The nutritional compositions of the present disclosure may also be formulated in product forms such as capsules, tablets, pills, caplets, gels, liquids (e.g., suspensions, solutions, emulsions, clear solutions), powders or other particulates, and so forth. These product forms generally contain only the essential ingredients as described herein, optionally in combination with other actives, processing aids or other dosage form excipients.

The nutritional products include liquid and spray dried powder forms, which includes ready-to-feed emulsified liquids and spray dried reconstitutable powders that can be diluted with water or other aqueous liquid to form a nutritional liquid prior to use.

Specifically, the nutritional composition may be human milk fortifiers for preterm infants intolerant to formulas based on cow milk proteins or soy proteins, human milk fortifiers for infants allergic to cow milk proteins or soy proteins, preterm infant formulas for preterm infants intolerant to formulas based on cow milk proteins or soy proteins, or preterm infant formulas for preterm infants allergic to cow milk proteins or soy proteins, infant formulas for infants intolerant to formulas based on cow milk proteins or soy proteins, infant formulas for infants allergic to cow milk proteins or soy proteins, hypoallergenic formulas for infants with food allergies generally, follow-on infant formulas for infants intolerant to formulas based on cow milk proteins or soy proteins, follow-on infant formulas for infants allergic to formulas based on cow milk proteins or soy proteins, toddler formulas for infants greater than six months of age who are intolerant to formulas based on cow milk proteins or soy proteins, toddler formulas for infants greater than six months of age who are allergic to formulas based on cow milk proteins or soy proteins, nutritional drinks for children and adults who are intolerant to nutritionals based on cow milk proteins or soy proteins, nutritional drinks for children and adults who are allergic to nutritionals based on cow milk proteins or soy proteins, nutritional drinks for geriatric patients who are intolerant to nutritionals based on cow milk proteins or soy proteins, nutritional drinks for geriatric patients who are allergic to nutritionals based on cow milk proteins or soy proteins, sports nutritional drinks for people who are intolerant to nutritionals based on cow milk proteins or soy proteins, sports nutritional drinks for people who are allergic to nutritionals based on cow milk proteins or soy proteins, sports nutritional drinks using hydrolyzed proteins with improved organoleptic properties, nutritional bars using hydrolyzed proteins with improved organoleptic properties, semi-elemental formulas for the nutritional needs of patients with malabsorption, maldigestion, and other gastrointestinal conditions, and formulas, bars, liquid or powdered protein supplements, or other food forms based on hydrolyzed proteins designed for patients who require hydrolyzed protein to manage their diets.

The nutritional compositions of the present disclosure, when formulated as a dietary product form, may potentially provide either a sole source or a supplemental source of nutrition to an individual. In this context, a sole source of nutrition is one that can be administered once or multiple times each day to potentially provide an individual with all or substantially all their fat, protein, carbohydrate, mineral, and vitamin needs per day or during the intended period of administration. A supplemental source of nutrition is defined herein as a dietary source that does not provide an individual with a potentially sole source of nutrition.

### Liquid Beverages

Liquid beverages include concentrated and ready-to-feed liquids. Non-limiting examples of liquid product forms suitable for use herein include liquid infant formulas (including both ready-to-feed formulations and concentrated formulations), liquid toddler formulas (including both ready-to-feed formulations and concentrated formulations), liquid human milk fortifiers (including both ready-to-feed and concentrated formulations), snack and meal replacement products, hot or cold beverages, carbonated or non carbonated beverages, juices or other acidified beverages, shakes, coffees, teas, enteral feeding compositions, and so forth. These liquid compositions are most typically formulated as suspensions or emulsions, but can also be formulated in any other suitable form such as clear liquid solutions, liquid gels, and so forth.

The nutritional liquid beverages of the present disclosure may be used as nutritional drinks for pre-term infants, infants, toddlers, children, and adults, including geriatric patients, who are intolerant or allergic to nutritionals based on cow milk proteins and/or soy proteins. The liquid beverages may also be used as semi-elemental formulas for the nutritional needs of patients with malabsorption, maldigestion, and other gastrointestinal conditions.

### A. Nutritional Emulsions

Nutritional emulsions comprise pea protein hydrolysate as the primary or sole source of protein and are aqueous emulsions further comprising fats and carbohydrates. These emulsions are flowable or drinkable liquids at from about 1°C to about 25°C and are typically in the form of oil-in-water, water-in-oil, or complex aqueous emulsions, although such emulsions are most typically in the form of oil-in-water emulsions having a continuous aqueous phase and a discontinuous oil phase.

The nutritional emulsions may be and typically are shelf stable. The nutritional emulsions typically contain up to about 95% by weight of water, including from about 50% to about 95%, also including from about 60% to about 90%, and also including from about 70% to about 85%, of water by weight of the nutritional emulsions.

The nutritional emulsions may be formulated with sufficient kinds and amounts of nutrients so as to provide a sole, primary, or supplemental source of nutrition, or to provide a specialized nutritional emulsion for use in individuals afflicted with specific diseases or conditions. For example, nutritional emulsions can be produced for use as infant, follow-on infant and toddler formulas, adult formulas and the like for users who are intolerant or allergic to nutritionals based on cow milk proteins and/or soy proteins. In another embodiment, the emulsions can be used as a hypoallergenic nutritional emulsion by individuals that have general food allergies. These nutritional emulsions may thus have a variety of product densities, but most typically have a density greater than about 1.03 g/ml, including greater than about 1.04 g/ml, including greater than about 1.055 g/ml, including from about 1.06 g/ml to about 1.12 g/ml, and also including from about 1.085 g/ml to about 1.10 g/ml.

The nutritional emulsions may have a caloric density tailored to the nutritional needs of the ultimate user, although in most instances the emulsions comprise from about 100 to about 500 kcal/240 ml, including from about 150 to about 350 kcal/240 ml, and also including from about 200 to about 320 kcal/240 ml.

In an alternative embodiment, the nutritional emulsion is a low-calorie nutritional emulsion and has a caloric density of about 6.5 kcal/fl oz (52.8 kcal/240 ml). In another embodiment, the nutritional emulsion has a caloric density of about 7.1 kcal/fl oz (57.6 kcal/240 ml). In one embodiment, the nutritional emulsion has a caloric density of about 7.7 kcal/fl oz (62.4 kcal/ml).

The nutritional emulsion may have a pH ranging from about 3.5 to about 8, but are most advantageously in a range of from about 4.5 to about 7.5, including from about 5.5 to about 7.3, including from about 6.2 to about 7.2.

Although the serving size for the nutritional emulsion can vary depending upon a number of variables, a typical serving size ranges from about 100 to about 300 ml, including from about 150 to about 250 ml, and including from about 190 ml to about 240 ml.

In one embodiment, the nutritional emulsion can be used as a human milk fortifier. In such embodiments, a typical single serving size includes about 5.0 ml.

### B. Substantially Clear Liquid Beverages

The substantially clear nutritional liquids of the present disclosure are thin liquids comprising at least protein, at least a portion of the proteins being pea protein hydrolysate, and carbohydrate as discussed below. The substantially clear nutritional liquids are substantially fat free; that is, the liquids are devoid of added fat except for that fat inherent to the raw materials or added fat at low concentrations to aid in the manufacture of the liquid. In this context, the term "fat free" means that the liquid typically contains less than 1.0 %, more typically less than 0.5%, and more typically less than 0.1 %, including zero percent, fat by weight of the nutritional liquid. These substantially clear nutritional liquids are flowable or drinkable liquids at from about 1°C to about 25°C.

The substantially clear nutritional liquids may be and typically are shelf-stable. The liquids typically contain up to about 95% by weight of water, including from about 50% to about 95%, also including from about 60% to about 90%, and also including from about 70% to about 85%, of water by weight of the substantially clear nutritional liquid.

The substantially clear nutritional liquids may be formulated with sufficient kinds and amounts of nutrients so as to provide a supplemental source of nutrition, or to provide a specialized nutritional liquid for use in individuals afflicted with specific diseases or conditions. For example, in one embodiment, the substantially clear nutritional liquid is a sports drink for use by users who are intolerant or allergic to nutritionals based on cow milk protein and/or soy proteins. In another embodiment, the substantially clear nutritional liquid is a sports drink including hydrolyzed protein with improved organoleptic properties. These substantially clear nutritional liquids may thus have a variety of product densities, but most typically have a density greater than about 1.040 g/ml, including from 1.06 g/ml to 1.12 g/ml, and also including from about 1.085 g/ml to about 1.10 g/ml.

The substantially clear nutritional liquids may have a caloric density tailored to the nutritional needs of the ultimate user, although in most instances the liquids comprise from about 90 to about 500 kcal/240 ml, including from about 150 to about 350 kcal/240 ml, including from about 180 to about 350 kcal/240 ml and also including from about 250 to about 320 kcal/240 ml. In other embodiments, the substantially clear nutritional liquids comprise from about 90 to about 500 kcal/480 ml, including from about 150 to about 350 kcal/480 ml, and also including from about 250 to about 320 kcal/480 ml.

The substantially clear nutritional liquids have a pH ranging from about 2.8 to about 4.6, including from about 2.9 to about 4.2, and also including from about 3.1 to about 3.9.

Although the serving size for the substantially clear nutritional liquid can vary depending upon a number of variables, a typical serving size ranges from about 100 to about 591 ml, including from about 150 to about 250 ml, including from about 190 ml to about 240 ml. Some specific serving sizes for the substantially clear nutritional liquid include 240 ml (8.1 ounce), 296 ml (10 ounce) and 480 ml (16 ounce).

### Nutritional Powders

The nutritional powders comprise pea protein hydrolysates as a primary or sole source of protein and are typically in the form of flowable or substantially flowable particulate compositions, or at least particulate compositions. Particularly suitable product forms include spray dried, agglomerated or dryblended powder compositions. The compositions can easily be scooped and measured with a spoon or similar other device, wherein the compositions can easily be reconstituted by the intended user with a suitable aqueous liquid, typically water, to form a nutritional formulation for immediate oral or enteral use. In this context, "immediate" use generally means within about 48 hours, most typically within about 24 hours, preferably right after reconstitution.

The nutritional powders may be formulated with sufficient kinds and amounts of nutrients so as to provide a sole, primary, or supplemental source of nutrition, or to provide a specialized nutritional powder for use in individuals afflicted with specific diseases or conditions. In one specific embodiment, the nutritional powder may be formulated for use with individuals who are intolerant or allergic to nutritionals based on cow milk proteins and/or soy proteins. In another embodiment, the emulsions can be used as hypoallergenic nutritional emulsions by individuals that have general food allergies.

The nutritional powders may be reconstituted with water prior to use to a caloric density tailored to the nutritional needs of the ultimate user, although in most instances the powders are reconstituted with water to form compositions comprising from about 100 to about 500 kcal/240 ml, including from about 150 to about 350 kcal/240 ml, and also including from about 200 to about 320 kcal/240 ml.

In an alternative embodiment, the nutritional powder may be reconstituted with water to form a low-calorie composition having a caloric density of about 52.8 kcal/240 ml. In another embodiment, the nutritional powder has a caloric density of about 57.6 kcal/240 ml. In one embodiment, the nutritional powder has a caloric density of about 62.4 kcal/240 ml.

Although the serving size for the reconstituted nutritional liquid can vary depending upon a number of variables, a typical serving size ranges from about 100 to about 300 ml, including from about 150 to about 250 ml, including from about 190 ml to about 240 ml.

In one specific embodiment, the reconstituted nutritional liquid can be used as a human milk fortifier. In such embodiments, a typical single serving size includes about 0.9 grams of powder per about 100 to about 300 ml of water.

### Other Solid Nutritional Products

The nutritional products can alternatively be in the form of solid nutritional bars and food products. For example, the nutritional products may be formulated as bars, sticks, or cookies, breads, cakes or other baked goods.

In another embodiment, the nutritional compositions of the present disclosure may also be formulated in product forms such as capsules, tablets, pills, caplets, and gels. These product forms generally contain only the essential ingredients as described herein, optionally in combination with other actives, processing aids or other dosage form excipients.

### Infant Formulas

When the nutritional product is an infant formula, the nutritional product may be a liquid or solid nutritional product as described above. The liquid or solid infant formula products of the present disclosure may have any caloric density suitable for the targeted infants, or provide such a density upon reconstitution of a powder embodiment or upon dilution of a liquid concentrate embodiment. For example, most common caloric densities for the infant formula embodiments of the present disclosure are generally at least about 19 kcal/fl oz (660 kcal/liter), more typically from about 20 kcal/fl oz (675-680 kcal/liter) to about 25 kcal/fl oz (820 kcal/liter), even more typically from about 20 kcal/fl oz (675-680 kcal/liter) to about 24 kcal/fl oz (800-810 kcal/liter). Generally, the 22-24 kcal/fl oz formulas are more commonly used in preterm or low birth weight infants, and the 20-21 kcal/fl oz (675-680 to 700 kcal/liter) formulas are more often used in term infants.

In an alternative embodiment, the infant formula is a low-calorie infant formula and has a caloric density of about 6.5 kcal/fl oz (220 kcal/liter). In another embodiment, the infant formula has a caloric density of about 7.1 kcal/fl oz (240 kcal/liter). In one embodiment, the infant formula has a caloric density of about 7.7 kcal/fl oz (260 kcal/liter).

Although total concentrations or amounts of the fat, protein, and carbohydrates may vary depending upon the product form (i.e., powder, ready-to-feed liquid, or concentrated liquid) and targeted dietary needs of the intended user, such concentrations or amounts most typically fall within one of the following embodied ranges, inclusive of any other essential fat, protein, and or carbohydrate ingredients as described herein.

For the liquid infant formula products, carbohydrate concentrations most typically range from about 5% to about 40%, including from about 7% to about 30%, including from about 10% to about 25%, by weight of the nutritional product; fat concentrations most typically range from about 1% to about 30%, including from about 2% to about 15%, and also including from about 4% to about 10%, by weight of the nutritional product; and protein concentrations most typically range from about 0.5% to about 30%, including from about 1% to about 15%, and also including from about 2% to about 10%, by weight of the nutritional product.

In an alternative embodiment, the liquid infant formula product is a low calorie liquid infant formula and includes less than about 5% by weight carbohydrate, about 1% by weight fat, and about 0.5% by weight protein.

The level or amount of carbohydrates, fats, and or proteins in the liquid nutritional products may also be characterized in addition to or in the alternative as a percentage of total calories in the nutritional compositions as set forth in the following table. These macronutrients for liquid nutritional products of the present disclosure are most typically formulated within any of the caloric ranges (embodiments A-F) described in the following table (each numerical value is preceded by the term "about").

| **Nutrient % Total Calories** | **Embodiment A** | **Embodiment B** | **Embodiment C** |
|---|---|---|---|
| **Carbohydrate** | 0-98 | 2-96 | 10-75 |
| **Protein** | 0-98 | 2-96 | 5-70 |
| **Fat** | 0-98 | 2-96 | 20-85 |

| | **Embodiment D** | **Embodiment E** | **Embodiment F** |
|---|---|---|---|
| **Carbohydrate** | 30-50 | 25-50 | 25-50 |
| **Protein** | 15-35 | 10-30 | 5-30 |
| **Fat** | 35-55 | 1-20 | 2-20 |

For example, liquid infant formulas (both ready-to-feed and concentrated liquids) include those embodiments in which the protein component may comprise from about 7.5% to about 25% of the caloric content of the formula; the carbohydrate component may comprise from about 35% to about 50% of the total caloric content of the infant formula; and the lipid component may comprise from about 30% to about 60% of the total caloric content of the infant formula. These ranges are provided as examples only, and are not intended to be limiting. Additional suitable ranges are noted in the following table (each numerical value is preceded by the term "about").

| **Nutrient % Total Calories** | **Embodiment G** | **Embodiment H** | **Embodiment I** |
|---|---|---|---|
| **Carbohydrates:** | 20-85 | 30-60 | 35-55 |
| **Lipid:** | 5-70 | 20-60 | 25-50 |
| **Protein:** | 2-75 | 5-50 | 7-40 |

When the nutritional product is a powdered infant formula, the protein component is present in an amount of from about 5% to about 35%, including from about 8% to about 12%, and including from about 10% to about 12% by weight of the powdered nutritional product; the fat component is present in an amount of from about 10% to about 35%, including from about 25% to about 30%, and including from about 26% to about 28% by weight of the powdered nutritional product; and the carbohydrate component is present in an amount of from about 30% to about 85%, including from about 45% to about 60%, including from about 50% to about 55% by weight of the powdered infant formula.

The total amount or concentration of fat, carbohydrate, and protein, in the powdered nutritional products of the present disclosure can vary considerably depending upon the selected formulation and dietary or medical needs of the intended user. Additional suitable examples of macronutrient concentrations are set forth below. In this context, the total amount or concentration refers to all fat, carbohydrate, and protein sources in the powdered product. For powdered infant formulas, such total amounts or concentrations are most typically and preferably formulated within any of the embodied ranges described in the following table (all numbers have "about" in front of them).

| **Nutrient (% Calories)** | **Embodiment A** | **Embodiment B** | **Embodiment C** |
|---|---|---|---|
| **Carbohydrate** | 20-85 | 30-60 | 35-55 |
| **Fat** | 5-70 | 20-60 | 25-50 |
| **Protein** | 2-75 | 5-50 | 7-40 |

### Pea Protein Hydrolysate

The nutritional products comprise pea protein hydrolysate as a primary or sole source of protein. In nutritional embodiments where a hypoallergenic composition is desired (such as a hypoallergenic infant formula), the nutritional composition will include 100% pea protein hydrolysate, by weight of the protein component. The pea protein hydrolysates used in the products of the present disclosure have been found to show lowered immunological reactivity and improved flavor and organoleptic properties as compared to other vegetable protein hydrolysates, such as soy proteins, as well as cow milk proteins. For example, as shown in FIG. 1, the pea protein hydrolysates for use in the nutritional products of the present disclosure have reduced levels of phytoestrogen (i.e., genistein and daidzein, which are isoflavones), aluminum, manganese, and phytates, thus improving the nutrient profile of the end nutritional product. Additionally, as described herein, the pea protein hydrolysate has a reduced antigen content and/or a reduced immunological reactivity. Thus, the pea protein hydrolysates provide a good source of protein for nutritional products that are designed for individuals who are intolerant and/or allergic to cow milk proteins and/or soy proteins, as well as to meet the nutritional needs of individuals with malabsorption, maldigestion, and other gastrointestinal conditions. These nutritional products further have improved flavor and emulsion stability.

The pea protein hydrolysates may be the sole source of protein in the nutritional products of the present disclosure. Alternatively, additional protein sources, as described below, may be used in the products in combination with the pea protein hydrolysates to provide the total protein. Accordingly, the pea protein hydrolysate provides at least 50% of the total protein in the nutritional product, including from about 50% to about 100% of the total protein, including from about 60% to about 95% of the total protein, including from about 70% to about 90% of the total protein, and also including from about 75% to about 85% of the total protein in the nutritional product. As noted herein, the additional protein sources may include partially hydrolyzed, hydrolyzed, and/or unhydrolyzed (intact) proteins.

Particularly, it has been surprisingly found that by controlling the hydrolysis of a pea protein source, such as pea protein isolate or pea protein concentrate, to a particular degree, pea protein hydrolysates can be produced with lowered antigen content, and thus lowered immunological reactivity, without adversely affecting the flavor attributes of the hydrolysate. The pea protein hydrolysates described herein are superior to previously known soy proteins as they have: (1) non-detectable levels of isoflavones; (2) decreased phytate, manganese, and aluminum concentrations; (3) improved organoleptic and flavor profiles; and (4) arc not genetically modified.

Generally, pea protein hydrolysates for use in the nutritional products of the present disclosure are produced by: (I) mixing a pea protein product having at least 65% protein (by weight dry matter) and water to form a slurry with a protein content of up to about 20%, preferably up to about 12%; (2) hydrolyzing the slurry; and (3) filtering the hydrolyzed slurry to obtain the protein hydrolysate.

The pea protein product used as raw material in step (1) can be any pea protein product, provided that the protein content thereof is at least 65% by weight dry matter, i.e. pea protein concentrates (with a protein content of above 70% by weight dry matter), or pea protein isolates (with a protein content of above 90% by weight dry matter). Commercially available pea protein concentrates and isolates are well known in the art.

The pea protein product is mixed with water to form a slurry with a protein content of up to about 20%, preferably up to about 12%. In one or more embodiments, the slurry may be heated prior to hydrolysis to further denaturing. For example, in one embodiment, the method further includes heating the slurry to a temperature above 60°C prior to hydrolysis. More suitably, the slurry can be heated to a temperature of from about 70°C to about 90°C for a period of from about 2 minutes to about 20 minutes prior to hydrolysis. In this manner, the protein is effectively denatured, and thus, the subsequent hydrolysis will generally proceed more rapidly. Further, by heating the slurry, the microbial stability during the hydrolysis, which can last for several hours, is secured effectively.

Once formed, and optionally heated, the temperature of the slurry is preferably adjusted and maintained at from about 40°C to about 70°C, including from about 50°C to about 60°C, prior to and during the hydrolysis reaction in accordance with methods known in the art. Of course, other heating steps and/or temperatures can be utilized as are known in the art.

Hydrolysis of the protein-containing slurry can be conducted enzymatically or non-enzymatically. In one embodiment, the protein-containing slurry is hydrolyzed to have an adjusted degree of hydrolysis of between about 7.0% and less than 19.0%, including between about 8.0% and about 16.0%, including between about 8.0% and about 13.5%, also including between about 12.0% and about 14.00%, and also including about 14.0% and a molecular weight distribution and antigen content as set forth below. Such hydrolysis, in one embodiment, may be conducted proteolytically as is known in the art.

In another embodiment, the hydrolysis is carried out with one or more unspecific acting endo- and/or exo-peptidase enzymes. Suitable unspecific acting endo- and/or exo-peptidase enzymes include, for example, enzymes derived from a strain of *Aspergillus,* in particular a strain of *Aspergillus niger,* a strain of *Aspergillus oryzae,* or a strain of *Aspergillus soyae,* or a strain of Bacillus, in particular a strain of *Bacillus amyloliquefaciens,* a strain of *Bacillus lentus,* a strain of *Bacillus licheniformis* or a strain of *Bacillus subtilis.*

Additional suitable endopeptidase enzymes for use in hydrolysis step (2) include, for example: (i) a glutamyl endopeptidase (EC 3.4.21.19); (ii) a lysyl endopeptidase (EC 3.4.21.50); (iii) a leucyl endopeptidase (EC 3.4.21.57); (iv) a glycyl endopeptidase (EC 3.4.22.25); (v) a prolyl endopeptidase (EC 3.4.21.26); (vi) trypsin (EC 3.4.21.4) or a trypsin-like (lysine/arginine specific) endopeptidase; or (vii) a peptidyl-Asp metalloendopeptidase (EC 3.4.24.33). Additional suitable exopeptidase enzymes include, for example, a carboxypeptidase, such as: (i) a proline carboxypeptidase (EC 3.4.16.2); (ii) a carboxypeptidase A (EC 3.4.17.1); (iii) a carboxypeptidase B (EC 3.4.17.2); (iv) a carboxypeptidase C (EC 3.4.16.5); (v) a carboxypeptidase D (EC 3.4.16.6); (vi) a lysine(arginine) carboxypeptidase (EC 3.4.17.3); (vii) a glycine carboxypeptidase (EC 3.4.17.4); (viii) an alanine carboxypeptidase (EC 3.4.17.6); (ix) a glutamate carboxypeptidase (EC 3.4.17.11); (x) a peptidyl-dipeptidase A (EC 3.4.1 5.1); or (xi) a peptidyl-dipeptidase (EC 3.4.15.5).

The exact amount of enzyme added to the slurry to obtain the desired characteristics of the pea protein hydrolysates will vary depending upon features such as: (1) the enzyme or enzyme system utilized; (2) the desired end degree of hydrolysis; and/or (3) the desired end molecular weight distribution. Because these are known parameters, one of ordinary skill in the art can easily determine the appropriate conditions to achieve the desired characteristics of the end pea protein hydrolysate. Typically, the enzyme or enzyme system is added to the slurry in amounts in the range of from about 10 mg to about 5000 g, including 35 mg to about 700 g per 35 kg of protein in the slurry.

The hydrolysis reaction will typically run for a desired length of time in order to obtain the desired degree of hydrolysis and/or the desired molecular weight profile. Typically, the hydrolysis reaction will continue for a period of from about 15 minutes to about 24 hours, including from about 30 minutes to about 240 minutes, and also including from about 45 minutes to about 120 minutes. Again, the exact amount of time required for the hydrolysis reaction will depend on specific features as noted above, but can be easily determined by one skilled in the art.

In other embodiments, the protein-containing slurry may be hydrolyzed using non-enzymatic means, such as by chemical and/or mechanical (physical) hydrolysis. Such technique was one also well known in the art.

Once the pea protein material has been hydrolyzed to the desired degree, the hydrolysis reaction is stopped, for example, by inactivating the enzyme or enzyme system, or by other conventional means. In one embodiment, the inactivation of the enzyme or enzyme system is carried out by heat treatment. This inactivation is especially well suited in cases where the pH of the final pea protein hydrolysate is to be relatively high.

In accordance with established practice, the enzyme preparation may suitably be inactivated by increasing the temperature of the incubation slurry to a temperature where the enzymes become inactivated, e.g. to above about 70°C, and suitably to a temperature of about 90°C.

Alternatively, the enzyme(s) can be inactivated by a suitable acid treatment. This inactivation is especially well suited in cases wherein the pH of the final pea protein hydrolysate is to be relatively low. For example, the enzyme(s) can be inactivated by decreasing the pH of the slurry to a value where the enzymes become inactivated, e.g. below about 4.0.

Optionally, after the hydrolysis reaction has been quenched, the method of preparation may further comprise treating the slurry with activated carbon for a suitable time period at a temperature of between about 50°C and about 70°C, for example, in an amount generally corresponding to between about I and about 5% carbon, calculated in relation to dry matter content. This optional carbon step may improve the color of the final pea protein hydrolysate and also further reduce the off-flavor of the pea protein hydrolysate.

Finally, the hydrolyzed slurry is filtered utilizing a suitable conventional filtering process to obtain the pea protein hydrolysate. For example, in one embodiment, a filtration is carried out by nanofiltration at a temperature between 50°C and 70°C and/or by evaporation, whereafter the retentate is collected as the pea protein hydrolysate solution. By means of the nanofiltration, a desalination can be carried out by proper selection of the membrane. Evaporation has the advantage of obtaining a high dry matter content in the concentrate before drying.

In one specific embodiment, the pea protein hydrolysate, once obtained by the filtration process outlined above, is subjected to a second filtration process, such an ultrafiltration process, to provide a molecular weight distribution suitable for use in semi-elemental and related nutritional products. The ultrafiltration unit can be a hollow-fiber membrane, such as for example, the commercially available unit under the trademark DiaFlo® from the Amicon Corporation and/or under the trademark Romicon® from the Romicon Corporation, having the desired nominal cutoff molecular weight values. The hollow-fiber membranes are packed within a filter cartridge to permit flow therethrough, whereby suitably small molecules pass through the membrane, the hydrolysate effluent rate being dependent upon the pressure drop across the membrane, and are taken in solution to a collecting zone, with the remainder of the solution, containing the higher molecular weight protein hydrolysate passing directly along the fibrous tubes, for recycle to the hydrolysis reactor or to a separate collecting zone, as desired.

Further, this optional second filtration step can reduce the resulting phytate levels in the pea protein hydrolysate. Particularly, the phytate content is reduced in the pea protein hydrolysates by at least about 10%, including at least about 15%, including at least about 20%, including at least about 25%, including at least about 30%, including at least about 35%, including at least about 40%, and including at least about 45% or more.

Pea protein hydrolysates for use in the nutritional products described herein are prepared using the above described methods and have the following characteristics. Exemplary pea protein hydrolysates made according to the above methods include those having a molecular weight distribution of less than 5% being greater than 5kD.

The pea protein hydrolysates produced according to the above-described methods additionally have reduced immunological reactivity. In one embodiment, the immunological reactivity of the pea protein hydrolysate is analyzed using inhibition ELISA (enzyme-linked immunosorbent assay) and is expressed as an amount of immunologically active antigen per amount of protein. For example, one gram of pea protein hydrolysate prepared accordingly to the methods described herein will typically include less than about 15,000 µg of immunologically active pea antigen, including less than about 10,000 µg of immunologically active pea antigen, including less than about 7,500 µg of immunologically active pea antigen, including less than about 5,000 µg of immunologically active pea antigen, including less than about 2,500 µg of immunologically active pea antigen, including less than about 500 µg of immunologically active pea antigen, including less than about 250 µg of immunologically active pea antigen, including less than about 100 µg of immunologically active pea antigen, including less than about 50 µg of immunologically active pea antigen, and including the pea protein hydrolysate being substantially free of immunologically active pea antigen.

The pea protein hydrolysate produced by the methods described above additionally have an immune response index of less than 2.5 log IRI units, including less than 2.4 log IRI units, including less than 2.3 log IRI units, including less than 2.2 log IRI units, including less than 2.1 log IRI units, and also including less than 2.0 log IRI units.

### Protein

The nutritional products of the present disclosure may optionally further comprise an additional source or sources of protein in addition to the pea protein hydrolysate. Any additional protein source that is suitable for use in oral nutritional products and is compatible with the essential elements and features of such products is suitable for use in combination with the pea protein hydrolysates.

When used in a liquid beverage, the total protein concentration in the nutritional liquid beverage may range from about 0.5% to about 40%, including from about 0.5% to about 30%, including from about 1% to about 15%, and also including from about 1% to about 10%, and also including from about 1% to about 7%, by weight of the nutritional liquid beverages. In one specific embodiment, the protein is present in a nutritional emulsion in an amount of about 6.0% by weight of the nutritional emulsion.

When used in solid nutritional products, such as nutritional powders, bars, and other food products, the total protein concentration in the solid nutritional products may range from about 1.0% to about 50%, including from about 10% to about 50%, and also including from about 10% to about 30%, by weight of the solid nutritional product. In one specific embodiment, the protein is present in a spray dried nutritional powder in an amount of about 19%, by weight of the spray dried nutritional powder.

Non-limiting examples of additional suitable protein or sources thereof for use in the nutritional products include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy) or combinations thereof. Non-limiting examples of such proteins include milk protein isolates, milk protein concentrates as described herein, casein protein isolates, whey protein, sodium or calcium caseinates, whole cow milk, partially or completely defatted milk, soy protein isolates, soy protein concentrates, and so forth.

### Carbohydrate

The nutritional products may further comprise any carbohydrates that are suitable for use in an oral nutritional product and are compatible with the essential elements and features of such products. Carbohydrate concentrations in the nutritional liquid beverages, for example, may range from about 5% to about 40%, including from about 7% to about 30%, including from about 10% to about 25%, by weight of the nutritional liquid beverage. In one specific embodiment, the carbohydrate is present in a nutritional emulsion in an amount of about 10.2%, by weight of the nutritional emulsion.

Carbohydrate concentrations in the solid nutritional products may range from about 10% to about 90%, including from about 20% to about 80%, further including from about 40% to about 60%, by weight of the solid nutritional product. In one specific embodiment, the carbohydrate is present in a spray dried nutritional powder in an amount of about 58%, by weight of the spray dried nutritional powder.

Non-limiting examples of suitable carbohydrates or sources thereof for use in the nutritional products described herein may include maltodextrin, hydrolyzed or modified starch or cornstarch, glucose polymers, corn syrup, corn syrup solids, rice-derived carbohydrates, pea-derived carbohydrates, potato-derived carbohydrates, tapioca, sucrose, glucose, fructose, lactose, high fructose corn syrup, honey, sugar alcohols (e.g., maltitol, erythritol, sorbitol), artificial sweeteners (e.g., sucralose, acesulfame potassium, stevia) and combinations thereof. A particularly desirable carbohydrate is a low dextrose equivalent (DE) maltodextrin.

### Fat

The nutritional products may further comprise fat, most typically as emulsified fat. The fat may be present in the nutritional liquid beverages in an amount of from 0% to about 30%, including 1% to about 30%, including from about 1% to about 20%, including from about 1% to about 15%, and also including from about 1.5% to about 5%, by weight of the nutritional emulsion. In one specific embodiment, a nutritional emulsion includes fat in an amount of about 1.6%, by weight of the nutritional emulsion.

It should be recognized by one skilled in the art that in some embodiments, such as in a substantially clear liquid beverage, the nutritional product will be substantially free of fat.

When used in solid nutritional products, the fat may be present in an amount of from about 1% to about 35%, including from about 1% to about 20%, including from about 1% to about 15%, and also including from about 5.0% to about 10%, by weight of the solid nutritional product. In one specific embodiment, a spray dried nutritional powder includes fat in an amount of about 7.5%, by weight of the spray dried nutritional powder.

Suitable sources of fat for use herein include any fat or fat source that is suitable for use in an oral nutritional product and is compatible with the essential elements and features of such products.

Non-limiting examples of suitable fats or sources thereof for use in the nutritional products described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

### Other Optional Ingredients

The nutritional products, including infant formulas, having the pea protein hydrolysate of the present disclosure may further comprise other optional components that may modify the physical, chemical, aesthetic or processing characteristics of the products or serve as pharmaceutical or additional nutritional components when used in the targeted population. Many such optional ingredients are known or otherwise suitable for use in medical food or other nutritional products or pharmaceutical dosage forms and may also be used in the compositions herein, provided that such optional ingredients are safe for oral administration and are compatible with the essential and other ingredients in the selected product form.

Non-limiting examples of such optional ingredients include preservatives, anti-oxidants, emulsifying agents, buffers, fructooligosaccharides, galactooligosaccharides, human milk oligosaccharides and other prebiotics, pharmaceutical actives, additional nutrients as described herein, colorants, flavors, thickening agents and stabilizers, emulsifying agents, lubricants, and so forth.

Examples of suitable HMOs that may be included in the compositions of the present disclosure include lacto-N-tetraose, lacto-N-neotetraose, lacto-N-fucopentaose I, lacto-N-fucopentaose II, lacto-N-fucopentaose III, lacto-N-fucopentaose V, lacto-N-hexaose, para-lacto-N-hexaose, lacto-N-neohexaose, para-lacto-N-neohexaose, monofucosyllacto-N-hexaose II, isomeric fucosylated lacto-N-hexaose (1), monofucosyllacto-N-hexaose, isomeric fucosylated lacto-N-hexaose (3), isomeric fucosylated lacto-N-hexaose (2), difucosyl-para-lacto-N-neohexaose, difucosyl-para-lacto-N-hexaose, difucosyllacto-N-hexaose, lacto-N-neoocataose, para-lacto-N-octanose, iso-lacto-N-octaose, lacto-N-octaose, monofucosyllacto-neoocataose, monofucosyllacto-N-ocataose, difucosyllacto-N-octaose I, difucosyllacto-N-octaose II, difucosyllacto-N-neoocataose II, difucosyllacto-N-neoocataose I, lacto-N-decaose, trifucosyllacto-N-neooctaose, trifucosyllacto-N-octaose, trifucosyl-iso-lacto-N-octaose, and combinations thereof. These HMOs are described more fully in U.S. Patent Application No. 2009/0098240, which is herein incorporated by reference in its entirety. Other suitable examples of HMOs that may be included in the compositions of the present disclosure include lacto-N-difucohexaose II, sialylα(2-3) lactose, sialylα(2-6) lactose, sialyl-lacto-N-tetraose a, sialyl-lacto-N-tetraose b, sialyl-lacto-N-tetraose c, sialyl-fucosyl-lacto-N-tetraose I, sialyl-fucosyl-lacto-N-tetraose II, and disialyl-lacto-N-tetraose.

The compositions may further comprise a sweetening agent, preferably including at least one sugar alcohol such as maltitol, erythritol, sorbitol, xylitol, mannitol, isolmalt, and lactitol, and also preferably including at least one artificial or high potency sweetener such as acesulfame K, aspartame, sucralose, saccharin, stevia, and tagatose. These sweetening agents, especially as a combination of a sugar alcohol and an artificial sweetener, are especially useful in formulating liquid beverage embodiments of the present disclosure having a desirable favor profile. These sweetener combinations are especially effective in masking undesirable flavors sometimes associated with the addition of vegetable proteins to a liquid beverage. Optional sugar alcohol concentrations in the nutritional product may range from at least about 0.01%, including from 0.1% to about 10%, and also including from about 1% to about 6%, by weight of the nutritional product. Optional artificial sweetener concentrations may range from about 0.01%, including from about 0.05% to about 5%, also including from about 0.1% to about 1.0%, by weight of the nutritional product.

A flowing agent or anti-caking agent may be included in the nutritional products such as the nutritional powders as described herein to retard clumping or caking of the powder over time and to make a powder embodiment flow easily from its container. Any known flowing or anti-caking agents that are known or otherwise suitable for use in a nutritional powder or product form are suitable for use herein, non limiting examples of which include tricalcium phosphate, silicates, and combinations thereof. The concentration of the flowing agent or anti-caking agent in the nutritional product varies depending upon the product form, the other selected ingredients, the desired flow properties, and so forth, but most typically range from about 0.1 % to about 4%, including from about 0.5% to about 2%, by weight of the nutritional product.

A stabilizer may also be included in the nutritional products. Any stabilizer that is known or otherwise suitable for use in a nutritional product is also suitable for use herein, some non-limiting examples of which include gums such as xanthan gum. The stabilizer may represent from about 0.1 % to about 5.0%, including from about 0.5% to about 3%, including from about 0.7% to about 1.5%, by weight of the nutritional product.

The compositions may further comprise any of a variety of other vitamins or related nutrients, non-limiting examples of which include vitamin A, vitamin D, vitamin E, vitamin K, thiamine, riboflavin, pyridoxine, vitamin B₁₂, carotenoids (e.g., beta-carotene, zeaxanthin, Iutein, lycopene), niacin, folic acid, pantothenic acid, biotin, vitamin C, choline, inositol, salts and derivatives thereof, and combinations thereof.

The compositions may further comprise any of a variety of other additional minerals, non-limiting examples of which include calcium, phosphorus, magnesium, iron, zinc, manganese, copper, sodium, potassium, molybdenum, chromium, chloride, and combinations thereof.

### Methods of Manufacture

The nutritional products of the present disclosure may be prepared by any known or otherwise effective manufacturing technique for preparing the selected product solid or liquid form. Many such techniques are known for any given product form such as nutritional liquids or powders or nutritional bars and can easily be applied by one of ordinary skill in the art to the nutritional compositions described herein.

The nutritional products of the present disclosure can therefore be prepared by any of a variety of known or otherwise effective formulation or manufacturing methods. In one suitable manufacturing process, for example, at least three separate slurries are prepared, including a protein-in-fat (PIF) slurry, a carbohydrate-mineral (CHO-MIN) slurry, a protein-in-water (PIW) slurry. The PIF slurry is formed by heating and mixing the oil (e.g., canola oil, corn oil, etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents (e.g. avicel, gellan, carrageenan). The resulting CHO-MIN slurry is held for 10 minutes with continued heat and agitation before adding additional minerals (e.g., potassium chloride, magnesium carbonate, potassium iodide, etc.), and/or carbohydrates (e.g., fructooligosaccharide, sucrose, corn syrup, etc.). The PIW slurry is then formed by mixing with heat and agitation the remaining protein, if any.

The resulting slurries are then blended together with heated agitation and the pH adjusted to 6.6-7.0, after which the composition is subjected to high-temperature short-time (HTST) processing during which the composition is heat treated, emulsified and homogenized, and then allowed to cool. Water soluble vitamins and ascorbic acid are added, the pH is adjusted to the desired range if necessary, flavors are added, and water is added to achieve the desired total solid level. The composition is then aseptically packaged to form an aseptically packaged nutritional emulsion. This emulsion can then be further diluted, heat-treated, and packaged to form a ready-to-feed or concentrated liquid, or it can be heat-treated and subsequently processed and packaged as a reconstitutable powder, e.g., spray dried, dry mixed, agglomerated.

When a substantially clear liquid beverage is desired, the above described manufacturing process is modified such that only a CHO-MIN slurry and a PIW slurry are blended to form the nutritional liquid beverage.

Other product forms such as nutritional bars may be manufactured, for example, using cold extrusion technology as is known and commonly described in the bar manufacturing art. To prepare such products, typically all of the powdered components are dry blended together, which typically includes any proteins, vitamin premixes, certain carbohydrates, and so forth. The fat-soluble components are then blended together and mixed with any powdered premixes. Finally any liquid components are then mixed into the composition, forming a plastic like composition or dough. The resulting plastic mass can then be shaped, without further physical or chemical changes occurring, by cold forming or extrusion, wherein the plastic mass is forced at relatively low pressure through a die, which confers the desired shape, The resultant exudate is then cut off at an appropriate position to give products of the desired weight. If desired, the solid product is then coated, to enhance palatability, and packaged for distribution.

Other suitable methods for making nutritional products are described, for example, in U.S. Pat. No. 6,365,218 (Borschel, et al.), U.S Patent 6,589,576 (Borschel, et al.), U.S. Pat. No. 6,306,908 (Carlson, et al.), U.S. Patent Application 20030118703 A1 (Nguyen, et al.), which descriptions are incorporated herein by reference to the extent that they are consistent herewith.

### Methods of Use

The nutritional products including pea protein hydrolysates as the sole source of protein are useful as a hypoallergenic nutrition source and/or a semi-elemental nutrition source for preterm infants, infants, toddlers, children, adults, and geriatrics that are intolerant and/or allergic to cow milk proteins, soy proteins, and/or have other general food allergies. Additionally, the nutritional products may be particularly suitable for use by individuals having malabsorption, maldigestion, and other gastrointestinal conditions.

In one embodiment, the nutritional products may be useful as providing a nutrition source using hydrolyzed proteins and having improved organoleptic properties, such as in sports drinks.

The nutritional products may be administered orally as needed to provide the desired level of nutrition, most typically in the form of one, two, three, four, five, six, seven, eight or more servings daily. Serving sizes may vary greatly and may range from about 1 to about 500 ml, including from about 5 to about 250 ml, including from about 10 to about 240 ml.

### EXAMPLE S

The following examples illustrate specific embodiments and/or features of the pea protein hydrolysates and/or nutritional products of the present disclosure. The examples are given solely for the purpose of illustration and are not to be construed as limitations of the present disclosure, as many variations thereof are possible without departing from the spirit and scope of the disclosure. All exemplified amounts are weight percentages based upon the total weight of the composition, unless otherwise specified.

The exemplified compositions are nutritional products prepared in accordance with manufacturing methods well known in the nutrition industry for preparing nutritional emulsions, other nutritional liquid beverages, nutritional powders, and/or nutritional bars.

### Example 1

In this Example, 10 samples of filtered (F) and non-filtered (N) pea protein hydrolysates prepared according to process as described herein are evaluated and compared to an intact pea protein isolate (ISO) sample for various physical characteristics. Filtered (F) indicates that the sample is subjected to the second filtration step described above. All samples are subjected to the first filtration step described above. The control sample (Intact) is not subjected to any hydrolysis procedure. Specifically, the samples are evaluated for degree of hydrolysis, molecular weight distribution, isoflavone content, and phytate content using standard analytical techniques. The results of the evaluations are shown in the table below.

| **Samples** | **Intact** | **AN** | **AF** | **BN** | **BF** | **CN** | **CF** | **DN** | **DF** | **EN** | **EF** |
|---|---|---|---|---|---|---|---|---|---|---|---|
| **Attribute** | | | | | | | | | | | |
| **% Nitrogen** | 12.90 | 13.30 | 13.60 | 13.20 | 14.00 | 12.90 | 13.60 | 13.10 | 14.00 | 12.90 | 13.90 |
| **% Protein** | 80.63 | 83.13 | 85 | 82.5 | 87.5 | 80.63 | 85 | 81.88 | 87.5 | 80.63 | 86.88 |

| **% Nitrogen X 6.25 from AN Proximates** | | | | | | | | | | | |
|---|---|---|---|---|---|---|---|---|---|---|---|
| Prot. from AA B+F% | 82.05 | 81.34 | 88.83 | 81.12 | 88.64 | 80.20 | 83.03 | 82.70 | 91.27 | 83.65 | 94.36 |
| Amino N | 1.12 | 1.68 | 2.05 | 2.2 | 2.39 | 2.88 | 2.98 | 2.95 | 3.31 | 3.41 | 3.97 |
| DH (% AN/TN) | 8.68 | 12.63 | 15.07 | 16.67 | 17.07 | 22.17 | 21.91 | 22.75 | 23.64 | 26.43 | 28.56 |
| Adjusted DH | 0.00 | 3.95 | 6.39 | 7.99 | 8.39 | 13.49 | 13.23 | 14.07 | 14.96 | 17.75 | 19.88 |
| | | | | | | | | | | | |
| Mg for 100 ml @ 5 mg protein/ml | 620 | 601 | 588 | 606 | 571 | 620 | 588 | 610 | 571 | 620 | 576 |
| Gm Par = 500 ml @ 2% Prot. | 12.4 | 12.02 | 11.75 | 12.12 | 11.42 | 12.4 | 11.76 | 12.2 | 11.42 | 12.4 | 11.52 |
| | | | | | | | | | | | |
| | | | | | | | | | | | |

| **MW Range by HPLC** | | | | | | | | | | | |
|---|---|---|---|---|---|---|---|---|---|---|---|
| >3 KD | 97.00% | 37.50% | 27.20% | 10.40% | 4.80% | 6.70% | 1.70% | 6.90% | 2.60% | 5.80% | 1.60% |
| > 5 KD | 93.30% | 26.30% | 16.50% | 4.10% | 1.00% | 2.70% | 0.40% | 2.50% | 0.60% | 2.00% | 0.40% |
| 3-5 KD | 3.70% | 11.20% | 10.70% | 6.30% | 3.80% | 4.00% | 1.30% | 4.40% | 2.00% | 3.80% | 1.20% |
| 1-3 KD | 1.00% | 28.80% | 34.50% | 31.30% | 33.10% | 18.10% | 17.80% | 22.90% | 24.60% | 18.40% | 19.20% |
| 0.5-1 KD | 0.40% | 20.30% | 23.60% | 30.40% | 33.20% | 29.30% | 31.80% | 29.40% | 31.60% | 28.10% | 30.30% |
| <0.5 KD | 1.60% | 13.40% | 14.70% | 27.90% | 28.90% | 45.90% | 48.70% | 40.80% | 41.20% | 47.70% | 48.90% |
| | | | | | | | | | | | |
| | | | | | | | | | | | |

| **Phytoestrogen Content** | | | | | | | | | | | |
|---|---|---|---|---|---|---|---|---|---|---|---|
| Daldzein mg/kg | < 2 | < 2 | < 2 | < 2 | < 2 | < 2 | < 2 | < 2 | < 2 | < 2 | < 2 |
| Genistein mg/kg | < 2 | < 2 | < 2 | < 2 | < 2 | < 2 | < 2 | < 2 | < 2 | < 2 | < 2 |
| Total Phos g/kg | 8.3 | 8.55 | 6.41 | 8.3 | 4.58 | 8.27 | 5.73 | 8.27 | 5.05 | 8.21 | 4.89 |
| Phytic Acid g/kg | 20.6 | 21.2 | 15.9 | 20.6 | 11.4 | 20.6 | 14.2 | 20.6 | 12.5 | 20.4 | 12.2 |

### Example 2

In this Example, the hypoallergenic potential of the pea protein hydrolysates analyzed in Example 1 are evaluated and compared to the intact pea protein isolate (ISO) sample of Example 1 using hyperimmunization of rabbits, in which rabbits are given multiple immunizations over a period of 35 days as described below. Immunogenicity of the pea protein hydrolysates is then tested by ELISA (enzyme-linked immunosorbent assay), which measures the rabbits' antigen-specific IgG response to the intact pea protein/pea protein hydrolysate immunogen.

The rabbits used in this Example are placed on a diet free of cow milk, soy, and pea proteins for fourteen days prior to the first immunization. At day 0, baseline antibody status of each of the rabbits is evaluated by blood sample from the rabbits. After obtaining baseline, each rabbit is immunized with 5.0 mg of pea protein equivalent amino acid content contained in 4.5 ml of antigen emulsified in Complete Freund's Adjuvant (CFA). The immunizations are delivered as follows: 20 X 0.1 ml per site/shaved flank intradermally, and 0.25 ml/popliteal lymph node area of the hind leg. On day 21, the second immunization is as follows: 0.50 ml/site X 2 sites/hind leg, intramuscularly; and, 0.25 ml/site X 4 sites subscapularly. For each immunization, a constant dose of 5 mg antigen amino acid equivalent is administered. Exsanguination of the rabbits is on study day 35.

A direct ELISA is used to quantitate antibody responses for each rabbit following hyperimmunization. Assay optimization is achieved using block titrations to determine antigen coating concentration and conjugate dilution. Following assay optimization, the pea protein-specific rabbit antibodies in the day 0 and day 35 serum samples are quantified. Additionally, an immune response index (IRI units) is determined by dividing the normalized day 35 antibody titer by the normalized baseline (day 0) titer of day 0 for each antiserum. Immunization groups contain 5-10 animals and the results for each animal are averaged. Comparisons of the antigen-specific IRI results predict hypoallergenic clinical performance. The results are provided in the table below:

| **Immune Response Index following Hyperimmunization (Normalized Day 35 titer/Normalized Day Zero titer)** | | | | | | | | | | | |
|---|---|---|---|---|---|---|---|---|---|---|---|
| | **Intact** | **AN** | **AF** | **BN** | **BF** | **CN** | **CF** | **DN** | **DF** | **EN** | **EF** |
| **IR1 Units** | 7155 | 9002 | 7558 | 330.8 | 167.8 | 167.2 | 10.73 | 921.4 | 932.8 | 1027 | 144.5 |
| **Log IRI Units** | 3.8546 | 3.9543 | 3.8784 | 2.5196 | 2.2245 | 2.2232 | 1.0307 | 2.9644 | 2.9698 | 3.0115 | 2.16 |

As shown in the table, the pea protein hydrolysates show a wide range of antibody response. The low reactivity may indicate that the pea protein hydrolysates still have epitopes that are recognized as antigenic, but not immunogenic by the rabbits. Moreover, pea protein hydrolysates BN, BF, CN, and CF showed a significantly lowered immunogenicity after hyperimmunization as compared to the other samples. The lowered immunogenicity of the pea protein hydrolysate samples indicates that the retained pea antigens do not have the capacity to stimulate an immune response.

### Example 3

In this Example, the hypoallergenic potential of the pea protein hydrolysates analyzed in Example 1 are evaluated and compared to the intact pea protein isolate (ISO) sample of Example 1 using antigen content, measured by inhibition ELISA. In this assay, intact pea protein is coated onto ELISA plates. Separately incubation tubes containing samples or pea protein solutions at standard concentrations plus anti-pea protein antibodies are prepared. After an incubation period aliquots of the sample (or standard) plus antibody solutions are added to the coated plates. Following incubation and washing an anti-rabbit IgG enzyme-conjugated antibody is added. The plates are incubated, washed, and an enzyme substrate is added. A color reaction occurs that is inversely proportional to the concentration of pea antigen in the sample which is quantified based on a standard curve. Results of this test for the 10 samples are shown in table below:

| **Immune Response Index following Hyperimmunization (Normalized Day 35 titer/ Normalized Day Zero titer)** | | | | | | | | | | | |
|---|---|---|---|---|---|---|---|---|---|---|---|
| | **Intact** | **AN** | **AF** | **BN** | **BF** | **CN** | **CF** | **DN** | **DF** | **EN** | **EF** |
| **Antigen µg/gm protein** | **1,000,000** | **493009** | **431210** | **55413** | **36007** | **15013** | **6417** | **18504** | **14900** | **14215** | **8644** |

### Example 4

In this Example, sensory evaluation is conducted for two bench screening sessions to characterize the taste of the pea protein hydrolysate samples of Example 1 retorted in both water and an Alimentum® matrix (Abbott Laboratories, Columbus, Ohio). The Alimentum® matrix contains carbohydrate, fat, vitamins, and minerals so as to simulate the biochemistry of a hypoallergenic formula. It is used to assess the mutual organoleptic effects on hydrolysate and finished formula on each other within the product matrix following retort treatment. The samples are compared to the current hydrolysate (casein hydrolysate) used in Alimentum® and the intact pea protein isolate (ISO) of Example 1. Particularly, it is known that hydrolyzed proteins are generally bitter, sour, salty, and/or beany. Thus, it is difficult to incorporate this functional ingredient into nutritional formulations without drastically impairing the taste of the product.

The samples are analyzed for intensity of various tastes, including for example: bitter, salt, sour, brothy, wet wool, acid sour, grassy/soybean, starchy, woody/sawdust, green/beany/dried peas, drying, powdery, oily, sulfidy/cabbage, burnt cheese, and gritty, Specifically, once the samples are prepared, four trained descriptive flavor panelists tasted each sample. After tasting, each panelist assessed the flavor attributes of the sample using a scoring system set forth in the table below and the results are then averaged.

| **Score** | **Description** |
|---|---|
| 1/2 | Very Slight |
| 1 | Slight |
| 1 1/2 | Slight to Moderate |
| 2 | Moderate |
| 2 1/2 | Moderate to Strong |
| 3 | Strong |

### Hydrolysates in water (2% solution)

| Basic Tastes and Aromatics | AF | BF | CF | DF | **EF** | Current Alimentum® Hydrolysate |
|---|---|---|---|---|---|---|
| Bitter | 1 | 1 ½ | 1 | 1 | 1 | 1 |
| Salt | 1 | 1 | ½ | ½ | 1 | |
| Sour | ½ | ½ | ½ | ½ | 1 | 1 |
| Brothy | 1 | 1 | 1 ½ | 1 ½ | 2 | 2 ½ |
| Wet Wool | 1 ½ | 1 ½ | 1 ½ | 1 ½ | 2 | 2 ½ |
| Acid Sour | | | | | | 1 |
| Appearance: | Translucent pale yellow | Translucent pale yellow | Translucent pale yellow | Translucent pale yellow | Translucent pale yellow | Translucent golden yellow |

### Non-Filtered Hydrolysates in water (2% solution)

| Basic Tastes and Aromatics | AN | BN | CN | DN | EN | ISO |
|---|---|---|---|---|---|---|
| Bitter | 1 | 1 | 1 ½ | 1 | 1 | ½ |
| Salt | | | | | 1 | ½ |
| Sour | 1 | 1 | 1 | 1 | 1 | 1 |
| Brothy | 1 | 1 | 1 | 1 | 1 ½ | |
| Acid Sour | | | | | 1 | |
| Grassy/soybe an | 2 | 2 | 1 | 1 ½ | 1 ½ | |
| Starchy | 1 | 1 | 1 | 1 | 1 | 1 |
| Woody/sawd ust | | | 2 | 1 | 1 | |
| Green/beany/ dried peas | | | | | | 1 ½ |
| Drying | 2 | 2 | 2 | 2 | 2 | 2 |
| Powdery mouthfeel | 1 ½ | 1 ½ | 1 ½ | 1 ½ | 1 ½ | 1 ½ |
| Appearance: | Cloudy yellow-tan | Cloudy yellow-tan | Cloudy yellow-tan | Cloudy yellow-tan | Cloudy yellow-tan | Opaque pale yellow-tan |

### Filtered Hydrolysates in Alimentum Matrix

| Basic Tastes and Aromatics | AF | BF | CF | DF | EF | Current Alimentum® Hydrolysate |
|---|---|---|---|---|---|---|
| Bitter | ½ | ½ | ½ | ½ | 1 | 1 |
| Salt | ½ | ½ | ½ | ½ | ½ | 1 |
| Sour | 1 | 1 | 1 | 1 | 1 | 1 |
| Sweet | 1 ½ | 1 ½ | 1 ½ | 1 ½ | 1 ½ | 1 ½ |
| Brothy | 2 turkey | 1 ½ | 1 ½ | 2 turkey | 2 turkey | 1 ½ |
| Acid Sour | 1 | 1 | 1 | 1 | 1 | 1 ½ |
| Drying | 1 | 1 | 1 | 1 | 1 | 1 |
| Oily Mouthfeel | 1 | 1 | 1 | 1 | 1 | 1 |
| Sulfidy/Cabb age | | | | | | 2 |
| Burnt Cheese | | | | | | 1 ½ |
| Wet Wool | | | | | | ½ |
| Appearance: | Opaque pale yellow | Opaque pale gray | Opaque pale gray | Opaque pale gray | Opaque pale gray | Opaque pale gray |

### Non-Filtered Hydrolysates in Alimentum Matrix

| Basic Tastes and Aromatics | AN* | BN* | CN* | DN* | EN* | ISO* |
|---|---|---|---|---|---|---|
| Bitter | 1 | 1 ½ | 1 ½ | 1 ½ | 1 ½ | ½ |
| Salt | ½ | ½ | ½ | ½ | ½ | ½ |
| Sour | 1 | 1 | 1 | 1 | 1 | 1 |
| Sweet | 1 ½ | 1 ½ | 1 ½ | 1 ½ | 1 ½ | 1 ½ |
| Brothy | 1 | 1 | 1 | 1 | 1 | |
| Acid Sour | 1 ½ | 2 | 2 | 2 | 2 | |
| Woody/Sawd ust | 1 ½ | 1 ½ | 1 ½ | 1 ½ | 1 ½ | |
| Drying | 1 | 1 | 1 | 1 | 1 | 1 |
| Powdery Mouthfeel | 1 ½ | 1 ½ | 1 ½ | 1 ½ | 1 ½ | |
| Gritty Mouthfeel | | | | | | 2 |
| Green/beany/ dried peas | | | | | | 2 |
| Appearance: | Opaque yellow-tan with gray | Opaque yellow-tan with gray | Opaque yellow-tan with gray | Opaque yellow-tan with gray | Opaque yellow-tan with gray | Opaque yellow-tan with gray |

### Examples 5-34

Examples 5-34 illustrate various infant formula nutritional powders of the present disclosure including pea protein hydrolysate as at least one source of protein, and in some cases the sole source of protein, the ingredients of which are listed in the table below. The pea protein hydrolysate has a degree of hydrolysis (adjusted) of from about 7.0% to less than 19.0%. These products are prepared by various methods in separate batches, are reconstituted with water prior to use to the desired target ingredient concentrations. The embodiments set forth below that include pea protein hydrolysate as the sole source of protein are hypoallergenic embodiments.

### Examples 5-9

| **Ingredient** | **Amounts per 22727.3 kg** | | | | |
|---|---|---|---|---|---|
| | **Example 5** | **Example 6** | **Example 7** | **Example 8** | **Example 9** |
| Corn Syrup | 9091.6 kg | 9091.6 kg | 8925.1 kg | 9091.6 kg | 8758.7 kg |
| Pea Protein Hydrolysate | 3329.4 kg | 3162.9 kg | 3495.9 kg | 2996.5 kg | 3662.3 kg |
| High Oleic Safflower Oil | 2608.1 kg | 2608.1 kg | 2608.1 kg | 2608.1 kg | 2608.1 kg |
| Sugar (Sucrose) | 2328.6 kg | 2495.1 kg | 2328.6 kg | 2661.5 kg | 2328.6 kg |
| Soy Oil | 1908.3 kg | 1908.3 kg | 1908.3 kg | 1908.3 kg | 1908.3 kg |
| Coconut Oil | 1844.7 kg | 1844.7 kg | 1844.7 kg | 1844.7 kg | 1844.7 kg |
| 5% Potassium Hydroxide | 600 kg | 600 kg | 600 kg | 600 kg | 600 kg |
| Potassium Citrate #1, #2 | 279.8 kg | 279.8 kg | 279.8 kg | 279.8 kg | 279.8 kg |
| Calcium Phosphate Tribasic | 252.5 kg | 252.5 kg | 252.5 kg | 252.5 kg | 252.5 kg |
| Calcium Phosphate Dibasic | 147.6 kg | 147.6 kg | 147.6 kg | 147.6 kg | 147.6 kg |
| Sodium Chloride | 76.2 kg | 76.2 kg | 76.2 kg | 76.2 kg | 76.2 kg |
| Magnesium Chloride | 58.8 kg | 58.8 kg | 58.8 kg | 58.8 kg | 58.8 kg |
| L-Methionine | 36.2 kg | 36.2 kg | 36.2 kg | 36.2 kg | 36.2 kg |
| Ascorbic Acid | 36.1 kg | 36.1 kg | 36.1 kg | 36.1 kg | 36.1 kg |
| Vitamin/Mineral/Taurine Premix | 32.5 kg | 32.5 kg | 32.5 kg | 32.5 kg | 32.5 kg |
| Choline Chloride | 11.5 kg | 11.5 kg | 11.5 kg | 11.5 kg | 11.5 kg |
| Vitamin A, D3, E, & K1 Premix | 8.7 kg | 8.7 kg | 8.7 kg | 8.7 kg | 8.7 kg |
| Ascorbyl Palmitate | 8.1 kg | 8.1 kg | 8.1 kg | 8.1 kg | 8.1 kg |
| Ferrous Sulfate | 6.9 kg | 6.9 kg | 6.9 kg | 6.9 kg | 6.9 kg |
| Potassium Chloride | 4.3 kg | 4.3 kg | 4.3 kg | 4.3 kg | 4.3 kg |
| Mixed Tocopherols | 3.7 kg | 3.7 kg | 3.7 kg | 3.7 kg | 3.7 kg |
| L-Carnitine | 2.5 kg | 2.5 kg | 2.5 kg | 2.5 kg | 2.5 kg |
| Beta-Carotene in vegetable oil | 28.3 g | 28.3 g | 28.3 g | 28.3 g | 28.3 g |
| Potassium Iodide | 22.9 g | 22.9 g | 22.9 g | 22.9 g | 22.9 g |

### Examples 10-14

| **Ingredient** | **Amounts per 22727.3 kg** | | | | |
|---|---|---|---|---|---|
| | **Example 10** | **Example 11** | **Example 12** | **Example 13** | **Example 14** |
| Corn Syrup | 9091.6 kg | 9091.6 kg | 9091.6 kg | 9091.6 kg | 9091.6 kg |
| Pea Protein Hydrolysate | 2996.5 kg | 2663.5 kg | 2330.6 kg | 1997.6 kg | 1664.7 kg |
| Soy Protein Isolate | 332.9 kg | 665.9 kg | 998.8 kg | 1331.8 kg | 1664.7 kg |
| High Oleic Safflower Oil | 2608.1 kg | 2608.1 kg | 2608.1 kg | 2608.1 kg | 2608.1 kg |
| Sugar (Sucrose) | 2328.6 kg | 2495.1 kg | 2328.6 kg | 2661.5 kg | 2328.6 kg |
| Soy Oil | 1908.3 kg | 1908.3 kg | 1908.3 kg | 1908.3 kg | 1908.3 kg |
| Coconut Oil | 1844.7 kg | 1844.7 kg | 1844.7 kg | 1844.7 kg | 1844.7 kg |
| 5% Potassium Hydroxide | 600 kg | 600 kg | 600 kg | 600 kg | 600 kg |
| Potassium Citrate #1, #2 | 279.8 kg | 279.8 kg | 279.8 kg | 279.8 kg | 279.8 kg |
| Calcium Phosphate Tribasic | 252.5 kg | 252.5 kg | 252.5 kg | 252.5 kg | 252.5 kg |
| Calcium Phosphate Dibasic | 147.6 kg | 147.6 kg | 147.6 kg | 147.6 kg | 147.6 kg |
| Sodium Chloride | 76.2 kg | 76.2 kg | 76.2 kg | 76.2 kg | 76.2 kg |
| Magnesium Chloride | 58.8 kg | 58.8 kg | 58.8 kg | 58.8 kg | 58.8 kg |
| L-Methionine | 36.2 kg | 36.2 kg | 36.2 kg | 36.2 kg | 36.2 kg |
| Ascorbic Acid | 36.1 kg | 36.1 kg | 36.1 kg | 36.1 kg | 36.1 kg |
| Vitamin/Mineral/Taurine Premix | 32.5 kg | 32.5 kg | 32.5 kg | 32.5 kg | 32.5 kg |
| Choline Chloride | 11.5 kg | 11.5 kg | 11.5 kg | 11.5 kg | 11.5 kg |
| Vitamin A, D3, E, & K1 Premix | 8.7 kg | 8.7 kg | 8.7 kg | 8.7 kg | 8.7 kg |
| Ascorbyl Palmitate | 8.1 kg | 8.1 kg | 8.1 kg | 8.1 kg | 8.1 kg |
| Ferrous Sulfate | 6.9 kg | 6.9 kg | 6.9 kg | 6.9 kg | 6.9 kg |
| Potassium Chloride | 4.3 kg | 4.3 kg | 4.3 kg | 4.3 kg | 4.3 kg |
| Mixed Tocopherols | 3.7 kg | 3.7 kg | 3.7 kg | 3.7 kg | 3.7 kg |
| L-Carnitine | 2.5 kg | 2.5 kg | 2.5 kg | 2.5 kg | 2.5 kg |
| Beta-Carotene in vegetable oil | 28.3 g | 28.3 g | 28.3 g | 28.3 g | 28.3 g |
| Potassium Iodide | 22.9 g | 22.9 g | 22.9 g | 22.9 g | 22.9 g |

### Examples 15-19

| **Ingredient** | **Amounts per 1000 kg** | | | | |
|---|---|---|---|---|---|
| | **Example 15** | **Example 16** | **Example 17** | **Example 18** | **Example 19** |
| Corn Syrup | 412.6 kg | 419.9 kg | 405.3 kg | 427.3 kg | 397.9 kg |
| Pea Protein Hydrolysate | 146.7 kg | 139.4 kg | 154.0 kg | 132.0 kg | 161.4 kg |
| High Oleic Safflower Oil | 114.7 kg | 114.7 kg | 114.7 kg | 114,7 kg | 114.7 kg |
| Sucrose | 97.9 kg | 97.9 kg | 97.9 kg | 97.9 kg | 97.9 kg |
| Soy Oil | 83.9 kg | 83.9 kg | 83.9 kg | 83.9 kg | 83.9 kg |
| Coconut Oil | 77.3 kg | 77.3 kg | 77.3 kg | 77.3 kg | 77.3 kg |
| Fructooligosaccharides | 16.2 kg | 16.2 kg | 16.2 kg | 16.2 kg | 16.2 kg |
| Potassium Citrate | 15.3 kg | 15.3 kg | 15.3 kg | 15.3 kg | 15.3 kg |
| Tricalcium Phosphate | 11.7 kg | 11.7 kg | 11.7 kg | 11.7 kg | 11.7 kg |
| Calcium Phosphate Dibasic | 6.8 kg | 6.8 kg | 6.8 kg | 6.8 kg | 6.8 kg |
| Sodium Chloride | 3.4 kg | 3.4 kg | 3.4 kg | 3.4 kg | 3.4 kg |
| Arachidonic Acid | 2.9 kg | 2.9 kg | 2.9 kg | 2.9 kg | 2.9 kg |
| Magnesium Chloride | 2.6 kg | 2.6 kg | 2.6 kg | 2.6 kg | 2.6 kg |
| L-Methionine | 1.7 kg | 1.7 kg | 1.7 kg | 1.7 kg | 1.7 kg |
| Ascorbic Acid | 1.6 kg | 1.6 kg | 1.6 kg | 1.6 kg | 1.6 kg |
| Vitamin/Mineral/Taurine Premix | 1.4 kg | 1.4 kg | 1.4 kg | 1.4 kg | 1.4 kg |
| Docosahexaenoic Acid | 1.1 kg | 1.1 kg | 1.1 kg | 1.1 kg | 1.1 kg |
| Choline Chloride | 507.7 g | 507.7 g | 507.7 g | 507.7 g | 507.7 g |
| Vitamin ADEK Premix | 372.6 g | 372.6 g | 372.6 g | 372.6 g | 372.6 g |
| Ascorbyl Palmitate | 357.5 g | 357.5 g | 357.5 g | 357.5 g | 357.5 g |
| Ferrous Sulfate | 305.3 g | 305.3 g | 305.3 g | 305.3 g | 305.3 g |
| Potassium Chloride | 198.5 g | 198.5 g | 198.5 g | 198.5 g | 198.5 g |
| Mixed Carotenoid Premix | 187.4 g | 187.4 g | 187.4 g | 187.4 g | 187.4 g |
| Mixed Tocopherols | 157.2 g | 157.2 g | 157.2 g | 157.2 g | 157.2 g |
| L-Carnitine | 107.8 g | 107.8 g | 107.8 g | 107.8 g | 107.8 g |
| Potassium Iodide | 1.0 g | 1.0 g | 1.0 g | 1.0 g | 1.0 g |
| 5% KOH | as needed | as needed | as needed | as needed | as needed |

### Examples 20-24

| **Ingredient** | **Amounts per 1000 kg** | | | | |
|---|---|---|---|---|---|
| | **Example 20** | **Example 21** | **Example 22** | **Example 23** | **Example 24** |
| Com Syrup | 412.6 kg | 412.6 kg | 412.6 kg | 412.6 kg | 412.6 kg |
| Pea Protein Hydrolysate | 132.0 kg | 117.4 kg | 102.7 kg | 88.0 kg | 73.35 kg |
| Soy Protein | 14.7 kg | 29.3 kg | 44.0 kg | 58.7 kg | 73.35 kg |
| High Oleic Safflower Oil | 114.7 kg | 114.7 kg | 114.7 kg | 114.7 kg | 114.7 kg |
| Sucrose | 97.9 kg | 97.9 kg | 97.9 kg | 97.9 kg | 97.9 kg |
| Soy Oil | 83.9 kg | 83.9 kg | 83.9 kg | 83.9 kg | 83.9 kg |
| Coconut Oil | 77.3 kg | 77.3 kg | 77.3 kg | 77.3 kg | 77.3 kg |
| Fructooligosaccharides | 16.2 kg | 16.2 kg | 16.2 kg | 16.2 kg | 16.2 kg |
| Potassium Citrate | 15.3 kg | 15.3 kg | 15.3 kg | 15.3 kg | 15.3 kg |
| Tricalcium Phosphate | 11.7 kg | 11.7 kg | 11.7 kg | 11.7 kg | 11.7 kg |
| Calcium Phosphate Dibasic | 6.8 kg | 6.8 kg | 6.8 kg | 6.8 kg | 6.8 kg |
| Sodium Chloride | 3.4 kg | 3.4 kg | 3.4 kg | 3.4 kg | 3.4 kg |
| Arachidonic Acid | 2.9 kg | 2.9 kg | 2.9 kg | 2.9 kg | 2.9 kg |
| Magnesium Chloride | 2.6 kg | 2.6 kg | 2.6 kg | 2.6 kg | 2.6 kg |
| L-Methionine | 1.7 kg | 1.7 kg | 1.7 kg | 1.7 kg | 1.7 kg |
| Ascorbic Acid | 1.6 kg | 1.6 kg | 1.6 kg | 1.6 kg | 1.6 kg |
| Vitamin/Mineral/Taurine Premix | 1.4 kg | 1.4 kg | 1.4 kg | 1.4 kg | 1.4 kg |
| Docosahexaenoic Acid | 1.1 kg | 1.1 kg | 1.1 kg | 1.1 kg | 1.1 kg |
| Choline Chloride | 507.7 g | 507.7 g | 507.7 g | 507.7 g | 507.7 g |
| Vitamin ADEK Premix | 372.6 g | 372.6 g | 372.6 g | 372.6 g | 372.6 g |
| Ascorbyl Palmitate | 357.5 g | 357.5 g | 357.5 g | 357.5 g | 357.5 g |
| Ferrous Sulfate | 305.3 g | 305.3 g | 305.3 g | 305.3 g | 305.3 |
| Potassium Chloride | 198.5 g | 198.5 g | 198.5 g | 198.5 g | 198.5 |
| Mixed Carotenoid Premix | 187.4 g | 187.4 g | 187.4 g | 187.4 g | 187.4 g |
| Mixed Tocopherols | 157.2 g | 157.2 g | 157.2 g | 157.2 g | 157.2 g |
| L-Carnitine | 107.8 g | 107.8 g | 107.8 g | 107.8 g | 107.8 g |
| Potassium Iodide | 1.0 g | 1.0 g | 1.0 g | 1.0 g | 1.0 g |
| 5% KOH | as needed | as needed | as needed | as needed | as needed |

### Examples 25-29

| **Ingredients** | **Amount per 22679.62 kg** | | | | |
|---|---|---|---|---|---|
| | **Example 25** | **Example 26** | **Example 27** | **Example 28** | **Example 29** |
| Maltodextrin | 7595 kg | 7793.9 kg | 7396.1 kg | 7992.8 kg | 7197.2 kg |
| Pea Protein Hydrolysate | 3978 kg | 3779.1 kg | 4176.9 kg | 3580.2 kg | 4375.8 kg |
| Sucrose | 3288 kg | 3288 kg | 3288 kg | 3288 kg | 3288 kg |
| High Oleic Safflower Oil | 2204 kg | 2204 kg | 2204 kg | 2204 kg | 2204 kg |
| Fractionated Coconut Oil (MCT) | 2162 kg | 2162 kg | 2162 kg | 2162 kg | 2162 kg |
| Soybean Oil | 1825 kg | 1825 kg | 1825 kg | 1825 kg | 1825 kg |
| Micronized Tricalcium Phosphate | 329 kg | 329 kg | 329 kg | 329 kg | 329 kg |
| Panodan FDPK | 326 kg | 326 kg | 326 kg | 326 kg | 326 kg |
| Potassium Citrate | 276 kg | 276 kg | 276 kg | 276 kg | 276 kg |
| Xanthan Gum, Extra Fine | 128 kg | 128 kg | 128 kg | 128 kg | 128 kg |
| Magnesium Chloride | 69.6 kg | 69.6 kg | 69.6 kg | 69.6 kg | 69.6 kg |
| Distilled Monoglycerides | 65.2 kg | 65.2 kg | 65.2 kg | 65.2 kg | 65.2 kg |
| Arachidonic Acid (AA) | 60.0 kg | 60.0 kg | 60.0 kg | 60.0 kg | 60.0 kg |
| Sodium Chloride | 59.2 kg | 59.2 kg | 59.2 kg | 59.2 kg | 59.2 kg |
| Ascorbic Acid | 51.2 kg | 51.2 kg | 51.2 kg | 51.2 kg | 51.2 kg |
| L-Cystine Dihydrochloride | 46.6 kg | 46.6 kg | 46.6 kg | 46.6 kg | 46.6 kg |
| Calcium Carbonate | 33.7 kg | 33.7 kg | 33.7 kg | 33.7 kg | 33.7 kg |
| Vitamin/Mineral/Taurine Premix | 31.1 kg | 31.1 kg | 31,1 kg | 31.1 kg | 31.1 kg |
| Docosahexaenoic Acid (DHA) | 22.5 kg | 22.5 kg | 22.5 kg | 22.5 kg | 22.5 kg |
| L-Tyrosine | 19.7 kg | 19.7 kg | 19.7 kg | 19.7 kg | 19.7 kg |
| Potassium Chloride | 16.7 kg | 16.7 kg | 16.7 kg | 16.7 kg | 16.7 kg |
| Choline Chloride | 13.0 kg | 13.0 kg | 13.0 kg | 13.0 kg | 13.0 kg |
| L-Tryptophan | 11.5 kg | 11.5 kg | 11.5 kg | 11.5 kg | 11.5 kg |
| Ferrous Sulfate | 10.9 kg | 10.9 kg | 10.9 kg | 10.9 kg | 10.9 kg |
| Vitamin A, D3, E, & K1 Premix | 8.53 kg | 8.53 kg | 8.53 kg | 8.53 kg | 8.53 kg |
| Ascorbyl Palmitate | 5.34 kg | 5.34 kg | 5.34 kg | 5.34 kg | 5.34 kg |
| Tocopherol-2 Food-Grade Antioxidant | 2.67 kg | 2.67 kg | 2.67 kg | 2.67 kg | 2.67 kg |
| L-Carnitine | 2.52 kg | 2.52 kg | 2.52 kg | 2.52 kg | 2.52 kg |
| Potassium Iodide | 33.8 g | 33.8 g | 33.8 g | 33.8 g | 33.8 g |
| 5% Potassium Hydroxide (processing aide) | as needed | as needed | as needed | as needed | as needed |

### Examples 30-34

| **Ingredients** | **Amount per 22679.62 kg** | | | | |
|---|---|---|---|---|---|
| | **Example 30** | **Example 31** | **Example 32** | **Example 33** | **Example 34** |
| Maltodextrin | 7595 kg | 7595 kg | 7595 kg | 7595 kg | 7595 kg |
| Pea Protein Hydrolysate | 3580.2 kg | 3182.4 kg | 2784.6 kg | 2386.8 kg | 1989 kg |
| Casein Hydrolysate | 397.8 kg | 795.6 kg | 1193.4 kg | 1591.2 kg | 1989 kg |
| Sucrose | 3288 kg | 3288 kg | 3288 kg | 3288 kg | 3288 kg |
| High Oleic Safflower Oil | 2204 kg | 2204 kg | 2204 kg | 2204 kg | 2204 kg |
| Fractionated Coconut Oil (MCT) | 2162 kg | 2162 kg | 2162 kg | 2162 kg | 2162 kg |
| Soybean Oil | 1825 kg | 1825 kg | 1825 kg | 1825 kg | 1825 kg |
| Micronized Tricalcium Phosphate | 329 kg | 329 kg | 329 kg | 329 kg | 329 kg |
| Panodan FDPK | 326 kg | 326 kg | 326 kg | 326 kg | 326 kg |
| Potassium Citrate | 276 kg | 276 kg | 276 kg | 276 kg | 276 kg |
| Xanthan Gum, Extra Fine | 128 kg | 128 kg | 128 kg | 128 kg | 128 kg |
| Magnesium Chloride | 69.6 kg | 69.6 kg | 69.6 kg | 69.6 kg | 69.6 kg |
| Distilled Monoglycerides | 65.2 kg | 65.2 kg | 65.2 kg | 65.2 kg | 65.2 kg |
| Arachidonic Acid (AA) | 60.0 kg | 60.0 kg | 60.0 kg | 60.0 kg | 60.0 kg |
| Sodium Chloride | 59.2 kg | 59.2 kg | 59.2 kg | 59.2 kg | 59.2 kg |
| Ascorbic Acid | 51.2 kg | 51.2 kg | 51.2 kg | 51.2 kg | 51.2 kg |
| L-Cystine Dihydrochloride | 46.6 kg | 46.6 kg | 46.6 kg | 46.6 kg | 46.6 kg |
| Calcium Carbonate | 33.7 kg | 33.7 kg | 33.7 kg | 33.7 kg | 33.7 kg |
| Vitamin/Mineral/Taurine Premix | 31.1 kg | 31.1 kg | 31.1 kg | 31.1 kg | 31.1 kg |
| Docosahexaenoic Acid (DHA) | 22.5 kg | 22.5 kg | 22.5 kg | 22.5 kg | 22,5 kg |
| L-Tyrosine | 19.7 kg | 19.7 kg | 19.7 kg | 19.7 kg | 19.7 kg |
| Potassium Chloride | 16.7 kg | 16.7 kg | 16.7 kg | 16.7 kg | 16.7 kg |
| Choline Chloride | 13.0 kg | 13.0 kg | 13.0 kg | 13.0 kg | 13.0 kg |
| L-Tryptophan | 11.5 kg | 11.5 kg | 11.5 kg | 11.5 kg | 11.5 kg |
| Ferrous Sulfate | 10.9 kg | 10.9 kg | 10.9 kg | 10.9 kg | 10.9 kg |
| Vitamin A, D3, E, & K1 Premix | 8.53 kg | 8.53 kg | 8.53 kg | 8.53 kg | 8.53 kg |
| Ascorbyl Palmitate | 5.34 kg | 5.34 kg | 5.34 kg | 5.34 kg | 5.34 kg |
| Tocopherol-2 Food-Grade Antioxidant | 2.67 kg | 2.67 kg | 2.67 kg | 2.67 kg | 2.67 kg |
| L-Carnitine | 2.52 kg | 2.52 kg | 2.52 kg | 2.52 kg | 2.52 kg |
| Potassium Iodide | 33.8 g | 33.8 g | 33.8 g | 33.8 g | 33.8 g |
| 5% Potassium Hydroxide (processing aide) | as needed | as needed | as needed | as needed | as needed |

### Examples 35-54

Examples 35-54 illustrate nutritional emulsion embodiments of the present disclosure including pea protein hydrolysate as at least one source of protein, and in some cases, the sole source of protein, the ingredients of which are listed in the table below. To prepare the nutritional emulsions, at least three separate slurries (e.g., CHO-MIN slurry, PIW slurry, PIF slurry) are prepared and then blended together, heat-treated, homogenized, and standardized. The resulting composition is aseptically packaged into plastic bottles or retort sterilized. The embodiments set forth below that include pea protein hydrolysates as the sole source of protein are hypoallergenic embodiments.

### Examples 35-39

| **Ingredient** | **Amount per 1000 Kg** | | | | |
|---|---|---|---|---|---|
| | **Example 35** | **Example 36** | **Example 37** | **Example 38** | **Example 39** |
| Ingredient Water | Q.S. | Q.S. | Q.S. | Q.S. | Q.S. |
| Corn Syrup | 63.5 kg | 63.5 kg | 63.5 kg | 63.5 kg | 63.5 kg |
| Pea Protein Hydrolysate | 18.9 kg | 17.9 kg | 19.8 kg | 17.0 kg | 20.8 kg |
| High Oleic Safflower Oil | 14.0 kg | 14.0 kg | 14.0 kg | 14.0 kg | 14.0 kg |
| Sucrose | 12.6 kg | 12.6 kg | 12.6 kg | 12.6 kg | 12.6 kg |
| Soy Oil | 10.5 kg | 10.5 kg | 10.5 kg | 10.5 kg | 10.5 kg |
| Coconut Oil | 10.0 kg | 10.0 kg | 10.0 kg | 10.0 kg | 10.0 kg |
| Fructooligosaccharides | 2.3 kg | 2.3 kg | 2.3 kg | 2.3 kg | 2.3 kg |
| Calcium Citrate | 1.7 kg | 1.7 kg | 1.7 kg | 1.7 kg | 1.7 kg |
| Micro Tricalcium Phosphate | 1.1 kg | 1.1 kg | 1.1 kg | 1.1 kg | 1.1 kg |
| Potassium Phosphate Monobasic | 744.4 g | 744.4 g | 744.4 g | 744.4 g | 744.4 g |
| Ascorbic Acid | 437.2 g | 437.2 g | 437.2 g | 437.2 g | 437.2 g |
| Potassium Citrate | 406.0 g | 406.0 g | 406.0 g | 406.0 g | 406.0 g |
| Magnesium Chloride | 373.4 | 373.4 | 373.4 | 373.4 | 373.4 |
| Arachidonic Acid Oil | 366.4 g | 366.4 g | 366.4 g | 366.4 g | 366.4 g |
| Monoglycerides | 350.0 g | 350.0 g | 350.0 g | 350.0 g | 350.0 g |
| Lecithin | 350.0 g | 350.0 g | 350.0 g | 350.0 g | 350.0 g |
| Sodium Chloride | 309.5 g | 309.5 g | 309.5 g | 309.5 g | 309.5 g |
| Potassium Phosphate Dibasic | 237.9 g | 237.9 g | 237.9 g | 237.9 g | 237.9 g |
| L-Methionine | 208.0 g | 208.0 g | 208.0 g | 208.0 g | 208.0 g |
| Seakem RLC | 200.1 g | 200.1 g | 200.1 g | 200.1 g | 200.1 g |
| Vitamin/Mineral/Taurine Premix | 176.4 g | 176.4 g | 176.4 g | 176.4 g | 176.4 g |
| Potassium Chloride | 143.6 g | 143.6 g | 143.6 g | 143.6 g | 143.6 g |
| Docosahexaenoic Acid Oil | 135.4 g | 135.4 g | 135.4 g | 135.4 g | 135.4 g |
| Choline Chloride | 71.3 g | 71.3 g | 71.3 g | 71.3 g | 71.3 g |
| Vitamin ADEK Premix | 48.0 g | 48.0 g | 48.0 g | 48.0 g | 48.0 g |
| Ferrous Sulfate | 44.8 g | 44.8 g | 44.8 g | 44.8 g | 44.8 g |
| Mixed Carotenoid Premix | 26.5 g | 26.5 g | 26.5 g | 26.5 g | 26.5 g |
| L-Carnitine | 13.8 g | 13.8 g | 13.8 g | 13.8 g | 13.8 g |
| Riboflavin | 2.0 g | 2.0 g | 2.0 g | 2.0 g | 2.0 g |
| Vitamin A Palmitate | 780.0 mg | 780.0 mg | 780.0 mg | 780.0 mg | 780.0 mg |
| Potassium Iodide | 150.0 mg | 150.0 mg | 150.0 mg | 150.0 mg | 150.0 mg |
| 5% KOH | as needed | as needed | as needed | as needed | as needed |

### Examples 40-44

| **Ingredient** | **Amount per 1000 Kg** | | | | |
|---|---|---|---|---|---|
| | **Example 40** | **Example 41** | **Example 42** | **Example 43** | **Example 44** |
| Ingredient Water | Q.S. | Q.S. | Q.S. | Q.S. | Q.S. |
| Corn Syrup | 63.5 kg | 63.5 kg | 63.5 kg | 63.5 kg | 63.5 kg |
| Pea Protein Hydrolysate | 17.0 kg | 15.1 kg | 13.2 kg | 11.3 kg | 9.45 kg |
| Soy Protein | 1.9 kg | 3.9 kg | 5.8 kg | 7.7 kg | 9.45 kg |
| High Oleic Safflower Oil | 14.0 kg | 14.0 kg | 14.0 kg | 14.0 kg | 14.0 kg |
| Sucrose | 12.6 kg | 12.6 kg | 12.6 kg | 12.6 kg | 12.6 kg |
| Soy Oil | 10.5 kg | 10.5 kg | 10.5 kg | 10.5 kg | 10.5 kg |
| Coconut Oil | 10.0 kg | 10.0 kg | 10.0 kg | 10.0 kg | 10.0 kg |
| Fructooligosaccharides | 2.3 kg | 2.3 kg | 2.3 kg | 2.3 kg | 2.3 kg |
| Calcium Citrate | 1.7 kg | 1.7 kg | 1.7 kg | 1.7 kg | 1.7 kg |
| Micro Tricalcium Phosphate | 1.1 kg | 1.1 kg | 1.1 kg | 1.1 kg | 1.1 kg |
| Potassium Phosphate Monobasic | 744.4 g | 744.4 g | 744.4 g | 744.4 g | 744.4 g |
| Ascorbic Acid | 437.2 g | 437.2 g | 437.2 g | 437.2 g | 437.2 g |
| Potassium Citrate | 406.0 g | 406.0 g | 406.0 g | 406.0 g | 406.0 g |
| Magnesium Chloride | 373.4 | 373.4 | 373.4 | 373.4 | 373.4 |
| Arachidonic Acid Oil | 366.4 g | 366.4 g | 366.4 g | 366.4 g | 366.4 g |
| Monoglycerides | 350.0 g | 350.0 g | 350.0 g | 350.0 g | 350.0 g |
| Lecithin | 350.0 g | 350.0 g | 350.0 g | 350.0 g | 350.0 g |
| Sodium Chloride | 309.5 g | 309.5 g | 309.5 g | 309.5 g | 309.5 g |
| Potassium Phosphate Dibasic | 237.9 g | 237.9 g | 237.9 g | 237.9 g | 237.9 g |
| L-Methionine | 208.0 g | 208.0 g | 208.0 g | 208.0 g | 208.0 g |
| Seakem RLC | 200.1 g | 200.1 g | 200.1 g | 200.1 g | 200.1 g |
| Vitamin/Mineral/Taurine Premix | 176.4 g | 176.4 g | 176.4 g | 176.4 g | 176.4 g |
| Potassium Chloride | 143.6 g | 143.6 g | 143.6 g | 143.6 g | 143.6 g |
| Docosahexaenoic Acid Oil | 135.4 g | 135.4 g | 135.4 g | 135.4 g | 135.4 g |
| Choline Chloride | 71.3 g | 71.3 g | 71.3 g | 71.3 g | 71.3 g |
| Vitamin ADEK Premix | 48.0 g | 48.0 g | 48.0 g | 48.0 g | 48.0 g |
| Ferrous Sulfate | 44.8 g | 44.8 g | 44.8 g | 44.8 g | 44.8 g |
| Mixed Carotenoid Premix | 26.5 g | 26.5 g | 26.5 g | 26.5 g | 26.5 g |
| L-Carnitine | 13.8 g | 13.8 g | 13.8 g | 13.8 g | 13.8 g |
| Riboflavin | 2.0 g | 2.0 g | 2.0 g | 2.0 g | 2.0 g |
| Vitamin A Palmitate | 780.0 mg | 780.0 mg | 780.0 mg | 780.0 mg | 780.0 mg |
| Potassium Iodide | 150.0 mg | 150.0 mg | 150.0 mg | 150.0 mg | 150.0 mg |
| 5% KOH | as needed | as needed | as needed | as needed | as needed |

### Examples 45-49

| **Ingredients** | **Amount per 45359.24 kg** | | | | |
|---|---|---|---|---|---|
| | **Example 45** | **Example 46** | **Example 47** | **Example 48** | **Example 49** |
| Ingredient Water | Q.S. | Q.S. | Q.S. | Q.S. | Q.S. |
| Sucrose | 2007 kg | 2007 kg | 2007 kg | 2007 kg | 2007 kg |
| Pea Protein Hydrolysate | 1025 kg | 973.8 kg | 1076.2 kg | 922.5 kg | 1127.5 kg |
| Modified Tapioca Starch | 989 kg | 989 kg | 989 kg | 989 kg | 989 kg |
| Safflower Oil | 644 kg | 644 kg | 644 kg | 644 kg | 644 kg |
| Fractionated Coconut Oil | 568 kg | 568 kg | 568 kg | 568 kg | 568 kg |
| Soy Oil | 479 kg | 479 kg | 479 kg | 479 kg | 479 kg |
| 5% KOH (processing aid) | 187 kg | 187 kg | 187 kg | 187 kg | 187 kg |
| Calcium Citrate | 69.9 kg | 69.9 kg | 69.9 kg | 69.9 kg | 69.9 kg |
| Calcium Phosphate Dibasic | 48.1 kg | 48.1 kg | 48.1 kg | 48.1 kg | 48.1 kg |
| Carrageenan | 45.4 kg | 45.4 kg | 45.4 kg | 45.4 kg | 45.4 kg |
| Potassium Phosphate Dibasic | 26.2 kg | 26.2 kg | 26.2 kg | 26.2 kg | 26.2 kg |
| Ascorbic Acid | 24.2 kg | 24.2 kg | 24.2 kg | 24.2 kg | 24.2 kg |
| Magnesium Chloride | 18.6 kg | 18.6 kg | 18.6 kg | 18.6 kg | 18.6 kg |
| M. Alpina Oil (ARA Oil) | 17.9 kg | 17.9 kg | 17.9 kg | 17.9 kg | 17.9 kg |
| Potassium Citrate | 15.9 kg | 15.9 kg | 15.9 kg | 15.9 kg | 15.9 kg |
| Sodium Chloride | 14.9 kg | 14.9 kg | 14.9 kg | 14.9 kg | 14.9 kg |
| Calcium Hydroxide | 14.0 kg | 14.0 kg | 14.0 kg | 14.0 kg | 14.0 kg |
| L-Cystine Dihydrochloride | 12.1 kg | 12.1 kg | 12.1 kg | 12.1 kg | 12.1 kg |
| Potassium Chloride | 11.2 kg | 11.2 kg | 11.2 kg | 11.2 kg | 11.2 kg |
| Water Soluble Vitamin Premix | 8.00 kg | 8.00 kg | 8.00 kg | 8.00 kg | 8.00 kg |
| *C. Cohnii Oil* (DHA Oil) | 6.71 kg | 6.71 kg | 6.71 kg | 6.71 kg | 6.71 kg |
| L-Tyrosine | 5.08 kg | 5.08 kg | 5.08 kg | 5.08 kg | 5.08 kg |
| L-Tryptophan | 4.67 kg | 4.67 kg | 4.67 kg | 4.67 kg | 4.67 kg |
| Choline Chloride | 3.36 kg | 3.36 kg | 3.36 kg | 3.36 kg | 3.36 kg |
| Oil Soluble Vitamin Premix | 2.37 kg | 2.37 kg | 2.37 kg | 2.37 kg | 2.37 kg |
| Ferrous Sulfate | 2.32 kg | 2.32 kg | 2.32 kg | 2.32 kg | 2.32 kg |
| L-Carnitine | 650 g | 650 g | 650 g | 650 g | 650 g |
| Riboflavin | 87.9 g | 87.9 g | 87.9 g | 87.9 g | 87.9 g |
| Vitamin A Palmitate | 17.6 g | 17.6 g | 17.6 g | 17.6 g | 17.6 g |
| Potassium Iodide | 8.70 g | 8.70 g | 8.70 g | 8.70 g | 8.70 g |

### Examples 50-54

| **Ingredients** | **Amount per 45359.24 kg** | | | | |
|---|---|---|---|---|---|
| | **Example 50** | **Example 51** | **Example 52** | **Example 53** | **Example 54** |
| Ingredient Water | Q.S. | Q.S. | Q.S. | Q.S. | Q.S. |
| Sucrose | 2007 kg | 2007 kg | 2007 kg | 2007 kg | 2007 kg |
| Pea Protein Hydrolysate | 922.5 kg | 820.0 kg | 717.5 kg | 615.0 kg | 512.5 kg |
| Casein Hydrolysate | 102.5 kg | 205.0 kg | 307.52 kg | 410.0 kg | 512.5 kg |
| Modified Tapioca Starch | 989 kg | 989 kg | 989 kg | 989 kg | 989 kg |
| Safflower Oil | 644 kg | 644 kg | 644 kg | 644 kg | 644 kg |
| Fractionated Coconut Oil | 568 kg | 568 kg | 568 kg | 568 kg | 568 kg |
| Soy Oil | 479 kg | 479 kg | 479 kg | 479 kg | 479 kg |
| 5% KOH (processing aid) | 187 kg | 187 kg | 187 kg | 187 kg | 187 kg |
| Calcium Citrate | 69.9 kg | 69.9 kg | 69.9 kg | 69.9 kg | 69.9 kg |
| Calcium Phosphate Dibasic | 48.1 kg | 48.1 kg | 48.1 kg | 48.1 kg | 48.1 kg |
| Carrageenan | 45.4 kg | 45.4 kg | 45.4 kg | 45.4 kg | 45.4 kg |
| Potassium Phosphate Dibasic | 26.2 kg | 26.2 kg | 26.2 kg | 26.2 kg | 26.2 kg |
| Ascorbic Acid | 24.2 kg | 24.2 kg | 24.2 kg | 24.2 kg | 24.2 kg |
| Magnesium Chloride | 18.6 kg | 18.6 kg | 18.6 kg | 18.6 kg | 18.6 kg |
| M. Alpina Oil (ARA Oil) | 17.9 kg | 17.9 kg | 17.9 kg | 17.9 kg | 17.9 kg |
| Potassium Citrate | 15.9 kg | 15,9 kg | 15.9 kg | 15.9 kg | 15.9 kg |
| Sodium Chloride | 14.9 kg | 14.9 kg | 14.9 kg | 14.9 kg | 14.9 kg |
| Calcium Hydroxide | 14.0 kg | 14.0 kg | 14.0 kg | 14.0 kg | 14.0 kg |
| L-Cystine Dihydrochloride | 12.1 kg | 12.1 kg | 12.1 kg | 12.1 kg | 12.1 kg |
| Potassium Chloride | 11.2 kg | 11.2 kg | 11.2 kg | 11.2 kg | 11.2 kg |
| Water Soluble Vitamin Premix | 8.00 kg | 8.00 kg | 8.00 kg | 8.00 kg | 8.00 kg |
| *C*. *Cohnii* Oil (DHA Oil) | 6.71 kg | 6.71 kg | 6.71 kg | 6.71 kg | 6.71 kg |
| L-Tyrosine | 5.08 kg | 5.08 kg | 5.08 kg | 5,08 kg | 5.08 kg |
| L-Tryptophan | 4.67 kg | 4.67 kg | 4.67 kg | 4.67 kg | 4.67 kg |
| Choline Chloride | 3.36 kg | 3.36 kg | 3.36 kg | 3.36 kg | 3.36 kg |
| Oil Soluble Vitamin Premix | 2.37 kg | 2.37 kg | 2.37 kg | 2.37 kg | 2.37 kg |
| Ferrous Sulfate | 2.32 kg | 2.32 kg | 2.32 kg | 2.32 kg | 2.32 kg |
| L-Carnitine | 650 g | 650 g | 650 g | 650 g | 650 g |
| Riboflavin | 87.9 g | 87.9 g | 87.9 g | 87.9 g | 87.9 g |
| Vitamin A Palmitate | 17.6 g | 17.6 g | 17.6 g | 17.6 g | 17.6 g |
| Potassium Iodide | 8.70 g | 8.70 g | 8.70 g | 8.70 g | 8.70 g |

### Examples 55-64

Examples 55-64 illustrate nutritional emulsions of the present disclosure, the ingredients of which are listed in the table below. To prepare the nutritional emulsions, at least three separate slurries (e.g., CHO-MIN slurry, PIW slurry, PIF slurry) are prepared and then blended together, heat-treated, homogenized, and standardized. The resulting composition is aseptically packaged into plastic bottles or retort sterilized. The embodiments set forth below that include pea protein hydrolysates as the sole source of protein are hypoallergenic embodiments.

| **Ingredients** | **Amounts (kg) per 1000 kg** | | | | |
|---|---|---|---|---|---|
| | **Example 55** | **Example 56** | **Example 57** | **Example 58** | **Example 59** |
| Water | Q.S | Q.S. | Q.S. | Q.S. | Q.S. |
| Maltodextrin | 120.0 | 120.0 | 120.0 | 120.0 | 120.0 |
| Sucrose | 7138 | 71.38 | 71.38 | 71.38 | 71.38 |
| Pea Protein Hydrolysate | 62.88 | 66.02 | 59.74 | 69.17 | 56.59 |
| Canola Oil | 27.5 | 27.5 | 27.5 | 27.5 | 27.5 |
| Corn Oil | 15.70 | 15.70 | 15.70 | 15.70 | 15.70 |
| Magnesium Phosphate | 1.92 | 1.92 | 1.92 | 1.92 | 1.92 |
| Potassium Citrate | 6.92 | 6.92 | 6.92 | 6.92 | 6.92 |
| Sodium Citrate | 0.903 | 0.903 | 0.903 | 0.903 | 0.903 |
| Lecithin | 1.50 | 1.50 | 1.50 | 1.50 | 1.50 |
| Sodium Tripolyphosphate | 1.06 | 1.06 | 1.06 | 1.06 | 1.06 |
| Potassium Phosphate dibasic | 0.730 | 0.730 | 0.730 | 0.730 | 0.730 |
| Potassium Chloride | 1.04 | 1.04 | 1.04 | 1.04 | 1.04 |
| Ascorbic Acid | 0.235 | 0.235 | 0.235 | 0.235 | 0.235 |
| Carrageenan | 0.150 | 0.150 | 0.150 | 0.150 | 0.150 |
| Potassium Hydroxide | 0.136 | 0.136 | 0.136 | 0.136 | 0.136 |
| TM/UTM Premix | 0.1684 | 0.1684 | 0.1684 | 0.1684 | 0.1684 |
| Gellan Gum | 0.050 | 0.050 | 0.050 | 0.050 | 0.050 |
| Vitamin A,D, E Premix | 0.0758 | 0.0758 | 0.0758 | 0.0758 | 0.0758 |
| Water sol. Vitamin premix | 0.0728 | 0.0728 | 0.0728 | 0.0728 | 0.0728 |
| Potassium Iodide | 0.00022 | 0.00022 | 0.00022 | 0.00022 | 0.00022 |
| Chromium Chloride | 0.000217 | 0.000217 | 0.000217 | 0.000217 | 0.000217 |
| Flavor | 3.3 | 3.3 | 3.3 | 3.3 | 3.3 |

| **Ingredients** | **Amounts (kg) per 1000 kg** | | | | |
|---|---|---|---|---|---|
| | **Example 60** | **Example 61** | **Example 62** | **Example 63** | **Example 64** |
| Water | Q.S | Q.S. | Q.S. | Q.S. | Q.S. |
| Maltodextrin | 120.0 | 120.0 | 120.0 | 120.0 | 120.0 |
| Sucrose | 71.38 | 71.38 | 71.38 | 71.38 | 71.38 |
| Milk Protein Concentrate | 9.33 | 7.46 | 5.60 | 3.73 | 1.87 |
| Canola Oil | 27.5 | 27.5 | 27.5 | 27.5 | 27.5 |
| Pea Protein Hydrolysate | 31.50 | 39.61 | 41.82 | 55.34 | 50.93 |
| Sodium Caseinate | 13.25 | 10.67 | 8.00 | 5.34 | 2.67 |
| Soy Protein Concentrate | 7.00 | 5.60 | 4.22 | 2.81 | 1.40 |
| Corn Oil | 15.70 | 15.70 | 15.70 | 15.70 | 15.70 |
| Whey Protein Concentrate | 1.75 | 1.40 | 1.05 | 0.70 | 0.35 |
| Magnesium Phosphate | 1.92 | 1.92 | 1.92 | 1.92 | 1.92 |
| Potassium Citrate | 6.92 | 6.92 | 6.92 | 6.92 | 6.92 |
| Sodium Citrate | 0.903 | 0.903 | 0.903 | 0.903 | 0.903 |
| Lecithin | 1.50 | 1.50 | 1.50 | 1.50 | 1.50 |
| Sodium Tripolyphosphate | 1.06 | 1.06 | 1.06 | 1.06 | 1.06 |
| Potassium Phosphate dibasic | 0.730 | 0.730 | 0.730 | 0.730 | 0.730 |
| Potassium Chloride | 1.04 | 1.04 | 1.04 | 1.04 | 1.04 |
| Ascorbic Acid | 0.235 | 0.235 | 0.235 | 0.235 | 0.235 |
| Carrageenan | 0.150 | 0.150 | 0.150 | 0.150 | 0.150 |
| Potassium Hydroxide | 0.136 | 0.136 | 0.136 | 0.136 | 0.136 |
| TM/UTM Premix | 0.1684 | 0.1684 | 0.1684 | 0.1684 | 0.1684 |
| Gellan Gum | 0.050 | 0.050 | 0.050 | 0.050 | 0.050 |
| Vitamin A,D, E Premix | 0.0758 | 0.0758 | 0.0758 | 0.0758 | 0.0758 |
| Water sol. Vitamin premix | 0.0728 | 0.0728 | 0.0728 | 0.0728 | 0.0728 |
| Potassium Iodide | 0.00022 | 0.00022 | 0.00022 | 0.00022 | 0.00022 |
| Chromium Chloride | 0.000217 | 0.000217 | 0.000217 | 0.000217 | 0.000217 |
| Flavor | 3.3 | 3.3 | 3.3 | 3.3 | 3.3 |

### Examples 65-74

Examples 65-74 illustrate substantially clear nutritional liquids of the present disclosure, the ingredients of which are listed in the table below. To prepare the nutritional liquids, at least two separate slurries (e.g., CHO-MIN slurry and PIW slurry) are prepared and then blended together, heat-treated, homogenized, and standardized. The resulting composition is aseptically packaged into plastic bottles or retort sterilized. The embodiments set forth below that include pea protein hydrolysates as the sole source of protein are hypoallergenic embodiments.

| **Ingredients** | **Amounts (kg) per 1000 kg** | | | | |
|---|---|---|---|---|---|
| | **Example 65** | **Example 66** | **Example 67** | **Example 68** | **Example 69** |
| Water | Q.S. | Q.S. | Q.S. | Q.S. | Q.S. |
| Sucrose | 109.0 | 109.0 | 0 | 54.5 | 54.5 |
| Isomaltulose | 0 | 0 | 109.0 | 54.5 | 54.5 |
| Pea Protein Hydrolysate | 42.90 | 45.05 | 40.76 | 47.19 | 38.61 |
| Phosphoric Acid | 2.0 | 2.0 | 2.0 | 2.0 | 2.0 |
| Citric Acid | 1.4 | 1.4 | 1.4 | 1.4 | 1.4 |
| N&A Apple Flavor | 700 g | 700 g | 700 g | 700 g | 700 g |
| Ascorbic Acid | 535 g | 535 g | 535 g | 535 g | 535 g |
| Caramel Liquid Color | 400 g | 400 g | 400 g | 400 g | 400 g |
| UTM/TM Premix | 230 g | 230 g | 230 g | 230 g | 230 g |
| Zinc Sulfate monohydrate* | 52.1 g | 52.1 g | 52.1 g | 52.1 g | 52.1 g |
| Ferrous Sulfate* | 40.8 g | 40.8 g | 40.8 g | 40.8 g | 40.8 g |
| Citric Acid* | 14.2 g | 14.2 g | 14.2 g | 14.2 g | 14.2 g |
| Manganese sulfate* | 13.0 | 13.0 | 13.0 | 13,0 | 13.0 |
| Copper Sulfate* | 7.1 g | 7.1 g | 7.1 g | 7.1 g | 7.1 g |
| Chromium Chloride* | 430 mg | 430 mg | 430 mg | 430 mg | 430 mg |
| Sodium Molybdate* | 339 mg | 339 mg | 339 mg | 339 mg | 339 mg |
| Sodium Selenate* | 146 mg | 146 mg | 146 mg | 146 mg | 146 mg |
| Water Dispersible ADEK Premix | 178 g | 178 g | 178 | 178 g | 178 g |
| di-Alpha-Tocopheryl Acetate** | 45.3 g | 45.3 g | 45.3 g | 45.3 g | 45.3 g |
| Vitamin A Palmitate** | 4.2 g | 4.2 g | 4.2 g | 4.2 g | 4.2 g |
| Phylloquinone** | 127 mg | 127 mg | 127 mg | 127 mg | 127 mg |
| Vitamin D3** | 23 mg | 23 mg | 23 mg | 23 mg | 23 mg |
| WSV Vitamin Premix | 37.9 g | 37.9 g | 37.9 g | 37.9 g | 37.9 g |
| Niacinamide*** | 14.2 g | 14.2 g | 14.2 g | 14.2 g | 14.2 g |
| d-Calcium pantothenate*** | 9.2 g | 9.2 g | 9.2 g | 9.2 g | 9.2 g |
| Thiamine chloride hydrochloride*** | 2.4 g | 2.4 g | 2.4 g | 2.4 g | 2.4 g |
| Pyridoxine hydrochloride*** | 2.3 g | 2.3 g | 2.3 g | 2.3 g | 2.3 g |

| | | | | | |
|---|---|---|---|---|---|
| *UTM/TM Premix; **ADEK Premix; ***WSV Vitamin Premix | | | | | |

| **Ingredients** | **Amounts (kg) per 1000 kg** | | | | |
|---|---|---|---|---|---|
| | **Example 70** | **Example 71** | **Example 72** | **Example 73** | **Example 74** |
| Water | Q.S. | Q.S. | Q.S. | Q.S. | Q.S. |
| Sucrose | 109.0 | 109.0 | 0 | 54.5 | 54.5 |
| Isomaltulose | 0 | 0 | 109.0 | 54.5 | 54.5 |
| Pea Protein Hydrolysate | 21.45 | 27.03 | 28.53 | 37.75 | 34.75 |
| Whey Protein Isolate | 18.59 | 12.87 | 11.15 | 6.24 | 3.44 |
| Hydrolyzed Casein | 2.86 | 4.29 | 1.72 | 2.14 | 0.75 |
| Phosphoric Acid | 2.0 | 2.0 | 2.0 | 2.0 | 2.0 |
| Citric Acid | 1.4 | 1.4 | 1.4 | 1.4 | 1.4 |
| N&A Apple Flavor | 700 g | 700 g | 700 g | 700 g | 700 g |
| Ascorbic Acid | 535 g | 535 g | 535 g | 535 g | 535 g |
| Caramel Liquid Color | 400 g | 400 g | 400 g | 400 g | 400 g |
| UTM/TM Premix | 230 g | 230 g | 230 g | 230 g | 230 g |
| Zinc Sulfate monohydrate* | 52.1 g | 52.1 g | 52.1 g | 52.1 g | 52.1 g |
| Ferrous Sulfate* | 40.8 g | 40.8 g | 40.8 g | 40.8 g | 40.8 g |
| Citric Acid* | 14.2 g | 14.2 g | 14.2 g | 14.2 g | 14.2 g |
| Manganese sulfate* | 13.0 | 13.0 | 13.0 | 13.0 | 13.0 |
| Copper Sulfate* | 7.1 g | 7.1 g | 7.1 g | 7.1 g | 7.1 g |
| Chromium Chloride* | 430 mg | 430 mg | 430 mg | 430 mg | 430 mg |
| Sodium Molybdate* | 339 mg | 339 mg | 339 mg | 339 mg | 339 mg |
| Sodium Selenate* | 146 mg | 146 mg | 146 mg | 146 mg | 146 mg |
| Water Dispersible ADEK Premix | 178 g | 178 g | 178 g | 178 g | 178 g |
| di-Alpha-Tocopheryl Acetate** | 45.3 g | 45.3 g | 45.3 g | 45.3 g | 45.3 g |
| Vitamin A Palmitate** | 4.2 g | 4.2 g | 4.2 g | 4.2 g | 4.2 g |
| Phylloquinone** | 127 mg | 127 mg | 127 mg | 127 mg | 127 mg |
| Vitamin D3** | 23 mg | 23 mg | 23 mg | 23 mg | 23 mg |
| WSV Vitamin Premix | 37.9 g | 37.9 g | 37.9 g | 37.9 g | 37.9 g |
| Niacinamide*** | 14.2 g | 14.2 g | 14.2 g | 14.2 g | 14.2 g |
| d-Calcium pantothenate*** | 9.2 g | 9.2 g | 9.2 g | 9.2 g | 9.2 g |
| Thiamine chloride hydrochloride*** | 2.4 g | 2.4 g | 2.4 g | 2.4 g | 2.4 g |
| Pyridoxine hydrochloride*** | 2.3 g | 2.3 g | 2.3 g | 2.3 g | 2.3 g |
| Riboflavin*** | 1.8 g | 1.8 g | 1.8 g | 1.8 g | 1.8 g |
| Folic Acid*** | 350 mg | 350 mg | 350 mg | 350 mg | 350 mg |
| Biotin*** | 277 mg | 277 mg | 277 mg | 277 mg | 277 mg |
| Cyanocobalamin*** | 6.3 mg | 6.3 mg | 6.3 mg | 6.3 mg | 6.3 mg |
| Folic Acid | 1.3 g | 1.3 g | 1.3 g | 1.3 g | 1.3g |
| Potassium Iodide | 204 mg | 204 mg | 204 mg | 204 mg | 204 mg |
| Beta-Alanine | 0 | 0 | 0 | 1.0 | 1.0 |

| | | | | | |
|---|---|---|---|---|---|
| *UTM/TM Premix; **ADEK Premix; ***WSV Vitamin Premix | | | | | |

### Examples 75-84

In Examples 75-84 illustrate concentrated liquid human milk fortifiers of the present disclosure, the ingredients of which are listed in the table below. To prepare the concentrated liquid human milk fortifiers, at least three separate slurries (e.g., CHO-MIN slurry and PIW slurry) are prepared and then blended together. The embodiments set forth below that include pea protein hydrolysates as the sole source of protein are hypoallergenic embodiments.

| **Ingredients** | **Amount per 1000 lbs** | | | | |
|---|---|---|---|---|---|
| | **Example 75** | **Example 76** | **Example 77** | **Example 78** | **Example 79** |
| Water | Q.S. | Q.S. | Q.S. | Q.S. | Q.S. |
| Pea Protein Hydrolysate | 104.6 lb | 99.4 lb | 109.8 lb | 94.1 lb | 115.1 lb |
| Maltodextrin | 93.0 lb | 93.0 lb | 93.0 lb | 93.0 lb | 93.0 lb |
| MCT Oil | 21.6 lb | 21.6 lb | 21.6 lb | 21.6 lb | 21.6 lb |
| Ultra Micronized Tricalcium Phosphate | 17.8 lb | 17.8 lb | 17.8 lb | 17.8 lb | 17.8 lb |
| Soy Oil | 12.9 lb | 12.9 lb | 12.9 lb | 12.9 lb | 12.9 lb |
| OSA-Modified Corn Starch | 12.0 lb | 12.0 lb | 12.0 lb | 12.0 lb | 12.0 lb |
| Coconut Oil | 7.9 lb | 7.9 lb | 7.9 lb | 7.9 lb | 7.9 lb |
| Potassium Citrate | 7.0 lb | 7.0 lb | 7.0 lb | 7.0 lb | 7.0 lb |
| Ascorbic Acid | 5.4 lb | 5.4 lb | 5.4 lb | 5.4 lb | 5.4 lb |
| Magnesium Chloride | 3.9 lb | 3.9 lb | 3.9 lb | 3.9 lb | 3.9 lb |
| ARA | 3.4 lb | 3.4 lb | 3.4 lb | 3.4 lb | 3.4 lb |
| L-Leucine | 2.8 lb | 2.8 lb | 2.8 lb | 2.8 lb | 2.8 lb |
| DHA | 2.6 lb | 2.6 lb | 2.6 lb | 2.6 lb | 2.6 lb |
| Potassium Chloride | 1.6 lb | 1.6 lb | 1.6 lb | 1.6 lb | 1.6 lb |
| L-Tyrosine | 1.4 lb | 1.4 lb | 1.4 lb | 1.4 lb | 1.4 lb |
| Distilled Monoglycerides | 1.0 lb | 1.0 lb | 1.0 lb | 1.0 lb | 1.0 lb |
| Nucleotide/Choline Premix | 399.0 g | 399.0 g | 399.0 g | 399.0 g | 399.0 g |
| M-Inositol | 296.0 g | 296.0 g | 296.0 g | 296.0 g | 296.0 g |
| Sodium Chloride | 246.3 g | 246.3 g | 246.3 g | 246.3 g | 246.3 g |
| L-Camitine | 147.0 g | 147.0 g | 147.0 g | 147.0 g | 147.0 g |
| Zinc Sulfate | 127.0 g | 127.0 g | 127.0 g | 127.0 g | 127.0 g |
| Niacinamide | 124.0 g | 124.0 g | 124.0 g | 124.0 g | 124.0 g |
| dl-Alpha-Tocopheryl Acetate | 114.0 g | 114.0 g | 114.0 g | 114.0 g | 114.0 g |
| Gellan Gum | 90.7 g | 90.7 g | 90.7 g | 90.7 g | 90.7 g |
| Ferrous Sulfate | 56.0 g | 56.0 g | 56.0 g | 56.0 g | 56.0 g |
| Choline Chloride | 48.0 g | 48.0 g | 48.0 g | 48.0 g | 48.0 g |
| Calcium Pantothenate | 39.3 g | 39.3 g | 39.3 g | 39.3 g | 39.3 g |
| Vitamin A Palmitate | 24.0 g | 24.0 g | 24.0 g | 24.0 g | 24.0 g |
| Riboflavin | 9.4 g | 9.4 g | 9.4 g | 9.4 g | 9.4 g |
| Vitamin D3 | 8.6 g | 8.6 g | 8.6 g | 8.6 g | 8.6 g |
| Copper Sulfate | 8.2 g | 8.2 g | 8.2 g | 8.2 g | 8.2 g |
| Pyridoxine Hydrochloride | 6.3 g | 6.3 g | 6.3 g | 6.3 g | 6.3 g |
| Thiamin Hydrochloride | 6.1 g | 6.1 g | 6.1 g | 6.1 g | 6.1 g |
| Lutein | 2.5 g | 2.5 g | 2.5 g | 2.5 g | 2.5 g |
| Beta Carotene | 1.7 g | 1.7 g | 1.7 g | 1.7 g | 1.7 g |
| Folic Acid | 0.88 g | 0.88 g | 0.88 g | 0.88 g | 0.88 g |
| Biotin | 0.74 g | 0.74 g | 0.74 g | 0.74 g | 0.74 g |
| Manganese Sulfate | 06.8 g | 06.8 g | 06.8 g | 06.8 g | 06.8 g |
| Phylloquinone | 0.31 g | 0.31 g | 0.31 g | 0.31 g | 0.31 g |
| Sodium Selenate | 0.06 g | 0.06 g | 0.06 g | 0.06 g | 0.06 g |
| Cyanocobalamin | 0.02 g | 0.02 g | 0.02 g | 0.02 g | 0.02 g |

| **Ingredients** | **Amount per 1000 lbs** | | | | |
|---|---|---|---|---|---|
| | **Example 80** | **Example 81** | **Example 82** | **Example 83** | **Example 84** |
| Water | Q.S. | Q.S. | Q.S. | Q.S. | Q.S. |
| Pea Protein Hydrolysate | 52.3 lb | 62.76 lb | 73.22 lb. | 83.68 lb | 94.14 lb |
| Calcium Hydrolysate | 52.3 lb | 41.84 lb | 31.38 lb | 20.92 lb | 10.46 lb |
| Maltodextrin | 93.0 lb | 93.0 lb | 93.0 lb | 93.0 lb | 93.0 lb |
| MCT Oil | 21.6 lb | 21.6 lb | 21.6 lb | 21.6 lb | 21.6 lb |
| Ultra Micronized Tricalcium Phosphate | 17.8 lb | 17.8 lb | 17.8 lb | 17.8 lb | 17.8 lb |
| Soy Oil | 12.9 lb | 12.9 lb | 12.9 lb | 12.9 lb | 12.9 lb |
| OSA-Modified Corn Starch | 12.0 lb | 12.0 lb | 12.0 lb | 12.0 lb | 12.0 lb |
| Coconut Oil | 7.9 lb | 7.9 lb | 7.9 lb | 7.9 lb | 7.9 lb |
| Potassium Citrate | 7.0 lb | 7.0 lb | 7.0 lb | 7.0 lb | 7.0 lb |
| Ascorbic Acid | 5.4 lb | 5.4 lb | 5.4 lb | 5.4 lb | 5.4 lb |
| Magnesium Chloride | 3.9 lb | 3.9 lb | 3.9 lb | 3.9 lb | 3.9 lb |
| ARA | 3.4 lb | 3.4 lb | 3.4 lb | 3.4 lb | 3.4 lb |
| L-Leucine | 2.8 lb | 2.8 lb | 2.8 lb | 2.8 lb | 2.8 lb |
| DHA | 2.6 lb | 2.6 lb | 2.6 lb | 2.6 lb | 2.6 lb |
| Potassium Chloride | 1.6 lb | 1.6 lb | 1.6 lb | 1.6 lb | 1.6 lb |
| L-Tyrosine | 1.4 lb | 1.4 lb | 1.4 lb | 1.4 lb | 1.4 lb |
| Distilled Monoglycerides | 1.0 lb | 1.0 lb | 1.0 lb | 1.0 lb | 1.0 lb |
| Nucleotide/Choline Premix | 399.0 g | 399.0 g | 399.0 g | 399.0 g | 399.0 g |
| M-Inositol | 296.0 g | 296.0 g | 296.0 g | 296.0 g | 296.0 g |
| Sodium Chloride | 246.3 g | 246.3 g | 246.3 g | 246.3 g | 246.3 g |
| L-Camitine | 147.0 g | 147.0 g | 147.0 g | 147.0 g | 147.0 g |
| Zinc Sulfate | 127.0 g | 127.0 g | 127.0 g | 127.0 g | 127.0 g |
| Niacinamide | 124.0 g | 124.0 g | 124.0 g | 124.0 g | 124.0 g |
| dl-Alpha-Tocopheryl Acetate | 114.0 g | 114.0 g | 114.0 g | 114.0 g | 114.0 g |
| Gellan Gum | 90.7 g | 90.7 g | 90.7 g | 90.7 g | 90.7 g |
| Ferrous Sulfate | 56.0 g | 56.0 g | 56.0 g | 56.0 g | 56.0 g |
| Choline Chloride | 48.0 g | 48.0 g | 48.0 g | 48.0 g | 48.0 g |
| Calcium Pantothenate | 39.3 g | 39.3 g | 39.3 g | 39.3 g | 39.3 g |
| Vitamin A Palmitate | 24.0 g | 24.0 g | 24.0 g | 24.0 g | 24.0 g |
| Riboflavin | 9.4 g | 9.4 g | 9.4 g | 9.4 g | 9.4 g |
| Vitamin D3 | 8.6 g | 8.6 g | 8.6 g | 8.6 g | 8.6 g |
| Copper Sulfate | 8.2 g | 8.2 g | 8.2 g | 8.2 g | 8.2 g |
| Pyridoxine Hydrochloride | 6.3 g | 6.3 g | 6.3 g | 6.3 g | 6.3 g |
| Thiamin Hydrochloride | 6.1 g | 6.1 g | 6.1 g | 6.1 g | 6.1 g |
| Lutein | 2.5 g | 2.5 g | 2.5 g | 2.5 g | 2.5 g |
| Beta Carotene | 1.7 g | 1.7 g | 1.7 g | 1.7 g | 1.7 g |
| Folic Acid | 0.88 g | 0.88 g | 0.88 g | 0.88 g | 0.88 g |
| Biotin | 0.74 g | 0.74 g | 0.74 g | 0.74 g | 0.74 g |
| Manganese Sulfate | 06.8 g | 06.8 g | 06.8 g | 06.8 g | 06.8 g |
| Phylloquinone | 0.31 g | 0.31 g | 0.31 g | 0.31 g | 0.31 g |
| Sodium Selenate | 0.06 g | 0.06 g | 0.06 g | 0.06 g | 0.06 g |
| Cyanocobalamin | 0.02 g | 0.02 g | 0.02 g | 0.02 g | 0.02 g |

### Examples 85-94

The following examples illustrate a human milk fortifier powder, which is added to human milk that may be administered to an infant in accordance with the methods of the present disclosure. Ingredients for manufacturing 8,172 kg of powdered human milk fortifier are listed in the table below.

| **Ingredients** | **Quantity to formulate 8,172 kg of fortifier** | | | | |
|---|---|---|---|---|---|
| | **Example 85** | **Example 86** | **Example 87** | **Example 88** | **Example 89** |
| Ingredient Water | 16,205 L | 16,205 L | 16,205 L | 16,205 L | 16,205 L |
| Corn syrup solids | 1603 kg | 1603 kg | 1603 kg | 1603 kg | 1603 kg |
| Magnesium chloride | 96.2 kg | 96.2 kg | 96.2 kg | 96.2 kg | 96.2 kg |
| Potassium citrate | 223.8 kg | 223.8 kg | 223.8 kg | 223.8 kg | 223.8 kg |
| Sodium citrate | 6.6 kg | 6.6 kg | 6.6 kg | 6.6 kg | 6.6 kg |
| Sodium chloride | 15.4 kg | 15.4 kg | 15.4 kg | 15.4 kg | 15.4 kg |
| MTC oil | 801 kg | 801 kg | 801 kg | 801 kg | 801 kg |
| Lecithin | 16.6 kg | 16.6 kg | 16.6 kg | 16.6 kg | 16.6 kg |
| Vitamin A | 2.36 kg | 2.36 kg | 2.36 kg | 2.36 kg | 2.36 kg |
| Vitamin D | 359.3 g | 359.3 g | 359.3 g | 359.3 g | 359.3 g |
| Vitamin K | 27.5 g | 27.5 g | 27.5 g | 27.5 g | 27.5 g |
| Tocopherol acetate | 15.8 kg | 15.8 kg | 15.8 kg | 15.8 kg | 15.8 kg |
| Calcium carbonate | 33.1 kg | 33.1 kg | 33.1 kg | 33.1 kg | 33.1 kg |
| Tricalcium phosphate | 646 kg | 646 kg | 646 kg | 616 kg | 646 kg |
| Pea Protein Hydrolysate | 2696.5 kg | 2561.7 kg | 2831.3 kg | 2426.9 kg | 2966.2 kg |
| Potassium citrate | 257.2 g | 257.2 g | 257.2 g | 257.2 g | 257.2 g |
| Ferrous sulfate | 3.7 kg | 3.7 kg | 3.7 kg | 3.7 kg | 3.7 kg |
| Zinc sulfate | 11.1 kg | 11.1 kg | 11.1 kg | 11.1 kg | 11.1 kg |
| Copper Sulfate | 1.84 kg | 1.84 kg | 1.84 kg | 1.84 kg | 1.84 kg |
| Manganese sulfate | 0.320 kg | 0.320 kg | 0.320 kg | 0.320 kg | 0.320 kg |
| Sodium selenate | 0.00 1 kg | 0.001 kg | 0.001 kg | 0.001 kg | 0.001 kg |
| Niacinamide | 0.98 kg | 0.98 kg | 0.98 kg | 0.98 kg | 0.98 kg |
| Riboflavin | 1.14 kg | 1.14 kg | 1.14 kg | 1.14 kg | 1.14 kg |
| Calcium pantothenate | 4.08 kg | 4.08 kg | 4.08 kg | 4.08 kg | 4.08 kg |
| Pyridoxine hydrochloride | 0.655 kg | 0.655 kg | 0.655 kg | 0.655 kg | 0.655 kg |
| m-inositol | 9.55 kg | 9.55 kg | 9.55 kg | 9.55 kg | 9.55 kg |
| Biotin | 0.0727 kg | 0.0727 kg | 0.0727 kg | 0.0727 kg | 0.0727 kg |
| Folic acid | 0.0775 kg | 0.0775 kg | 0.0775 kg | 0.0775 kg | 0.0775 kg |
| Cyanocobalamin | 0.0016 kg | 0.0016 kg | 0.0016 kg | 0.0016 kg | 0.0016 kg |
| Ascorbic acid | 153.5 kg | 153.5 kg | 153.5 kg | 153.5 kg | 153.5 kg |

| **Ingredients** | **Quantity to formulate 8,172 kg of fortifier** | | | | |
|---|---|---|---|---|---|
| | **Example 90** | **Example 91** | **Example 92** | **Example 93** | **Example 94** |
| Ingredient Water | 16,205 L | 16,205 L | 16,205 L | 16,205 L | 16,205 L |
| Corn syrup solids | 1603 kg | 1603 kg | 1603 kg | 1603 kg | 1603 kg |
| Magnesium chloride | 96.2 kg | 96.2 kg | 96.2 kg | 96.2 kg | 96.2 kg |
| Potassium citrate | 223.8 kg | 223.8 kg | 223.8 kg | 223.8 kg | 223.8 kg |
| Sodium citrate | 6.6 kg | 6.6 kg | 6.6 kg | 6.6 kg | 6.6 kg |
| Sodium chloride | 15.4 kg | 15.4 kg | 15.4 kg | 15.4 kg | 15.4 kg |
| MTC oil | 801 kg | 801 kg | 801 kg | 801 kg | 801 kg |
| Lecithin | 16.6 kg | 16.6 kg | 16.6 kg | 16.6 kg | 16.6 kg |
| Vitamin A | 2.36 kg | 2.36 kg | 2.36 kg | 2.36 kg | 2.36 kg |
| Vitamin D | 359.3 g | 359.3 g | 359.3 g | 359.3 g | 359.3 g |
| Vitamin K | 27.5 g | 27.5 g | 27.5 g | 27.5 g | 27.5 g |
| Tocopherol acetate | 15.8 kg | 15.8 kg | 15.8 kg | 15.8 kg | 15.8 kg |
| Calcium carbonate | 33.1 kg | 33.1 kg | 33.1 kg | 33.1 kg | 33.1 kg |
| Tricalcium phosphate | 646 kg | 646 kg | 646 kg | 646 kg | 646 kg |
| Pea Protein Hydrolysate | 1348.3 kg | 1537.0 kg | 1981.9 kg | 1941.5 kg | 2669.58 kg |
| Soy protein concentrate | 753.0 kg | 602.4 kg | 451.8 kg | 301.2 kg | 150.6 kg |
| Non fat dry milk | 1653.5 kg | 1322.8 kg | 992.1 kg | 661.4 kg | 3.31 kg |
| Potassium citrate | 257.2 g | 257.2 g | 257.2 g | 257.2 g | 257.2 g |
| Ferrous sulfate | 3.7 kg | 3.7 kg | 3.7 kg | 3.7 kg | 3.7 kg |
| Zinc sulfate | 11.1 kg | 11.1 kg | 11.1 kg | 11.1 kg | 11.1 kg |
| Copper sulfate | 1.84 kg | 1.84 kg | 1.84 kg | 1.84 kg | 1.84 kg |
| Manganese sulfate | 0.320 kg | 0.320 kg | 0.320 kg | 0.320 kg | 0.320 kg |
| Sodium selenate | 0.001 kg | 0.001 kg | 0.001 kg | 0.001 kg | 0.001 kg |
| Niacinamide | 0.98 kg | 0.98 kg | 0.98 kg | 0.98 kg | 0.98 kg |
| Riboflavin | 1.14 kg | 1.14 kg | 1.14 kg | 1.14 kg | 1.14 kg |
| Calcium pantothenate | 4.08 kg | 4.08 kg | 4.08 kg | 4.08 kg | 4.08 kg |
| Pyridoxine Hydrochloride | 0.655 kg | 0.655 kg | 0.655 kg | 0.655 kg | 0.655 kg |
| m-inositol | 9.55 kg | 9.55 kg | 9.55 kg | 9.55 kg | 9.55 kg |
| Biotin | 0.0727 kg | 0.0727 kg | 0.0727 kg | 0.0727 kg | 0.0727 kg |
| Folic acid | 0.0775 kg | 0.0775 kg | 0.0775 kg | 0.0775 kg | 0.0775 kg |
| Cyanocobalamin | 0.0016 kg | 0.0016 kg | 0.0016 kg | 0.0016 kg | 0.0016 kg |
| Ascorbic acid | 153.5 kg | 153.5 kg | 153.5 kg | 153.5 kg | 153.5 kg |

### Examples 95-104

In these Examples, a gelled human milk fortifier is prepared in accordance with the present disclosure. The ingredients for the gelled human milk fortifier are shown in the following table.

The gelled human milk fortifier is prepared by solubilizing and combining ingredients into a homogeneous aqueous mixture which is subjected to an adequate heat treatment to achieve long term shelf stability.

To begin the manufacturing process, the ingredients that supply the macronutrients (carbohydrate, protein, fat and minerals) are combined in multiple slurries together and with water. This blend is subjected to an initial heat treatment and then tested to verify proper nutrient levels.

| **Ingredients** | **Quantity to formulate 32,000 lbs of fortifier** | | | | |
|---|---|---|---|---|---|
| | **Example 95** | **Example 96** | **Example 97** | **Example 98** | **Example 99** |
| Water | Q.S. | Q.S. | Q.S. | Q.S. | Q.S. |
| Corn Maltodextrin | 1450 kg | 1450 kg | 1450 kg | 1450 kg | 1450 kg |
| Corn Syrup Solids | 1388 kg | 1388 kg | 1388 kg | 1388 kg | 1388 kg |
| Medium Chain triglycerides | 694 kg | 694 kg | 694 kg | 694 kg | 694 kg |
| Pea Protein Hydrolysate | 1944.4 kg | 1847.18 kg | 2041.6 kg | 1750.0 kg | 2138.8 kg |
| Calcium Phosphate | 271 kg | 271 kg | 271 kg | 271 kg | 271 kg |
| Ascorbic Acid | 152 kg | 152 kg | 152 kg | 152 kg | 152 kg |
| Magnesium Chloride | 38.0 kg | 38.0 kg | 38.0 kg | 38.0 kg | 38.0 kg |
| Potassium Citrate | 12.2 kg | 12.2 kg | 12.2 kg | 12.2 kg | 12.2 kg |
| Sodium Chloride | 12.1 kg | 12.1 kg | 12.1 kg | 12.1 kg | 12.1 kg |
| Soy Lecithin | 8.84 kg | 8.84 kg | 8.84 kg | 8.84 kg | 8.84 kg |
| M-Inositol | 7.98 kg | 7.98 kg | 7.98 kg | 7.98 kg | 7.98 kg |
| Magnesium Phosphate | 5.55 kg | 5.55 kg | 5.55 kg | 5.55 kg | 5.55 kg |
| M. Alpina Oil | 5.35 kg | 5.35 kg | 5.35 kg | 5.35 kg | 5.35 kg |
| Niacinamide | 4.35 kg | 4.35 kg | 4.35 kg | 4.35 kg | 4.35 kg |
| Alpha-Tocopheryl Acetate | 4.21 kg | 4.21 kg | 4.21 kg | 4.21 kg | 4.21 kg |
| Zinc Sulfate | 3.51 kg | 3.51 kg | 3.51 kg | 3.51 kg | 3.51 kg |
| C. Cohnii Oil | 3.45 kg | 3.45 kg | 3.45 kg | 3.45 kg | 3.45 kg |
| Choline Chloride | 2.90 kg | 2.90 kg | 2.90 kg | 2.90 kg | 2.90 kg |
| Calcium Pantothenate | 1.89 kg | 1.89 kg | 1.89 kg | 1.89 kg | 1.89 kg |
| Potassium Phosphate | 1.62 kg | 1.62 kg | 1.62 kg | 1.62 kg | 1.62 kg |
| Ferrous Sulfate | 1.64 kg | 1.64 kg | 1.64 kg | 1.64 kg | 1.64 kg |
| Vitamin A Palmitate | 900 g | 900 g | 900 g | 900 g | 900 g |
| Cupric Sulfate | 678 g | 678 g | 678 g | 678 g | 678 g |
| Riboflavin | 572 g | 572 g | 572 g | 572 g | 572 g |
| Thiamine Hydrochloride | 373 g | 373 g | 373 g | 373 g | 373 g |
| Pyridoxine Hydrochloride | 232 g | 232 g | 232 g | 232 g | 232 g |
| Vitamin D3 | 152 g | 0.0775 kg | 0.0775 kg | 0.0775 kg | 0.0775 kg |
| Folic Acid | 47.9 g | 0.0016 kg | 0.0016 kg | 0.0016 kg | 0.0016 kg |
| Biotin | 34.1 g | 153.5 kg | 153.5 kg | 153.5 kg | 153.5 kg |
| Manganese Sulfate | 23.2 g | 23.2 g | 23.2 g | 23.2 g | 23.2 g |
| Phylloquinone | 11.6 g | 11.6 g | 11.6 g | 11.6 g | 11.6 g |
| Cyanocobalamin | 1.60 g | 1.60 g | 1.60 g | 1.60 g | 1.60 g |
| Sodium Selenate | 0.798 g | 0.798 g | 0.798 g | 0.798 g | 0.798 g |
| Calcium Carbonate | as needed | as needed | as needed | as needed | as needed |
| Sodium Citrate | as needed | as needed | as needed | as needed | as needed |
| Potassium Hydroxide | as needed | as needed | as needed | as needed | as needed |

| **Ingredients** | **Quantity to formulate 32,000 lbs of fortifier** | | | | |
|---|---|---|---|---|---|
| | **Example 100** | **Example 101** | **Example 102** | **Example 103** | **Example 104** |
| Water | Q.S. | Q.S. | Q.S. | Q.S. | Q.S. |
| Condensed Skim Milk | 2625 kg | 2100 kg | 1575 kg | 1050 kg | 525 kg |
| Non-Fat Milk Solids | 682.5 kg | 546 kg | 409.5 kg | 273 kg | 136.5 kg |
| Corn Maltodextrin | 1450 kg | 1450 kg | 1450 kg | 1450 kg | 1450 kg |
| Corn Syrup Solids | 1388 kg | 1388 kg | 1388 kg | 1388 kg | 1388 kg |
| Medium Chain triglycerides | 694 kg | 694 kg | 694 kg | 694 kg | 694 kg |
| Pea Protein Hydrolysate | 972.2 kg | 1166.6 kg | 1361.1 kg | 1555.5 kg | 1750.0 kg |
| Whey Protein Concentrate | 317 kg | 253.6 kg | 190.2 kg | 126.8 kg | 63.4 kg |
| Calcium Phosphate | 271 kg | 271 kg | 271 kg | 271 kg | 271 kg |
| Ascorbic Acid | 152 kg | 152 kg | 152 kg | 152 kg | 152 kg |
| Magnesium Chloride | 38.0 kg | 38.0 kg | 38.0 kg | 38.0 kg | 38.0 kg |
| Potassium Citrate | 12.2 kg | 12.2 kg | 12.2 kg | 12.2 kg | 12.2 kg |
| Sodium Chloride | 12.1 kg | 12.1 kg | 12.1 kg | 12.1 kg | 12.1 kg |
| Soy Lecithin | 8.84 kg | 8.84 kg | 8.84 kg | 8.84 kg | 8.84 kg |
| M-Inositol | 7.98 kg | 7.98 kg | 7.98 kg | 7.98 kg | 7.98 kg |
| Magnesium Phosphate | 5.55 kg | 5.55 kg | 5.55 kg | 5.55 kg | 5.55 kg |
| M. Alpina Oil | 5.35 kg | 5.35 kg | 5.35 kg | 5.35 kg | 5.35 kg |
| Niacinamide | 4.35 kg | 4.35 kg | 4.35 kg | 4.35 kg | 4.35 kg |
| Alpha-Tocopheryl Acetate | 4.21 kg | 4.21 kg | 4.21 kg | 4.21 kg | 4.21 kg |
| Zinc Sulfate | 3.51 kg | 3.51 kg | 3.51 kg | 3.51 kg | 3.51 kg |
| C. Cohnii Oil | 3.45 kg | 3.45 kg | 3.45 kg | 3.45 kg | 3.45 kg |
| Choline Chloride | 2.90 kg | 2.90 kg | 2.90 kg | 2.90 kg | 2.90 kg |
| Calcium Pantothenate | 1.89 kg | 1.89 kg | 1.89 kg | 1.89 kg | 1.89 kg |
| Potassium Phosphate | 1.62 kg | 1.62 kg | 1.62 kg | 1.62 kg | 1.62 kg |
| Ferrous Sulfate | 1.64 kg | 1.64 kg | 1.64 kg | 1.64 kg | 1.64 kg |
| Vitamin A Palmitate | 900 g | 900 g | 900 g | 900 g | 900 g |
| Cupric Sulfate | 678 g | 678 g | 678 g | 678 g | 678 g |
| Riboflavin | 572 g | 572 g | 572 g | 572 g | 572 g |
| Thiamine Hydrochloride | 373 g | 373 g | 373 g | 373 g | 373 g |
| Pvridoxine Hydrochloride | 232 g | 232 g | 232 g | 232 g | 232 g |
| Vitamin D3 | 152 g | 0.0775 kg | 0.0775 kg | 0.0775 kg | 0.0775 kg |
| Folic Acid | 47.9 g | 0.0016 kg | 0.0016 kg | 0.0016 kg | 0.0016 kg |
| Biotin | 34.1 g | 153.5 kg | 153.5 kg | 153.5 kg | 153.5 kg |
| Manganese Sulfate | 23.2 g | 23.2 g | 23.2 g | 23.2 g | 23.2 g |
| Phylloquinone | 11.6 g | 11.6 g | 11.6 g | 11.6 g | 11.6 g |
| Cyanocobalamin | 1.60 g | 1.60 g | 1.60 g | 1.60 g | 1.60 g |
| Sodium Selenate | 0.798 g | 0.798 g | 0.798 g | 0.798 g | 0.798 g |
| Calcium Carbonate | as needed | as needed | as needed | as needed | as needed |
| Sodium Citrate | as needed | as needed | as needed | as needed | as needed |
| Potassium Hydroxide | as needed | as needed | as needed | as needed | as needed |

### Examples 105-114

Examples 105-114 illustrate nutritional powders of the present disclosure, the ingredients of which are listed in the table below. These products are prepared by spray drying methods in separate batches, arc reconstituted with water prior to use to the desired target ingredient concentrations. All ingredient amounts are listed as kg per 1000 kg batch of product, unless otherwise specified.

| **Ingredients** | **Amounts (kg) per 1000 kg** | | | | |
|---|---|---|---|---|---|
| | **Example 105** | **Example 106** | **Example 107** | **Example 108** | **Example 109** |
| **Maltrin** | 436.7 | 436.7 | 436.7 | 436.7 | 436.7 |
| **Sucrose** | 145.5 | 145.5 | 145.5 | 145.5 | 145.5 |
| **Pea Protein Hydrolysate** | 186.8 | 177.5 | 196.14 | 168.12 | 205.5 |
| **Conjugated Linoleic Acid** | 67.0 | 63.65 | 70.35 | 60.3 | 73.7 |
| **FOS Powder** | 33.6 | 33.6 | 33.6 | 33.6 | 33.6 |
| **High oleic sunflower oil** | 33.1 | 33.1 | 33.1 | 33.1 | 33.1 |
| **Calcium β-hydroxy-β-methylbutarate** | 31.6 | 31.6 | 31.6 | 31.6 | 31.6 |
| **Canola Oil** | 28.3 | 28.3 | 28.3 | 28.3 | 28.3 |
| **Soy Oil** | 13.3 | 13.3 | 13.3 | 13.3 | 13.3 |
| **Potassium Citrate** | 10.3 | 10.3 | 10.3 | 10.3 | 10.3 |
| **Sodium Citrate** | 5.8 | 5.8 | 5.8 | 5.8 | 5.8 |
| **Potassium Chloride** | 5.2 | 5.2 | 5.2 | 5.2 | 5.2 |
| **Magnesium Chloride** | 4.7 | 4.7 | 4.7 | 4.7 | 4.7 |
| **Potassium hydroxide** | 3.1 | 3.1 | 3.1 | 3.1 | 3.1 |
| **Sodium phosphate dibasic dehydrate** | 3.0 | 3.0 | 3.0 | 3.0 | 3.0 |
| **Sodium chloride** | 2.5 | 2.5 | 2.5 | 2.5 | 2.5 |
| **Choline Chloride** | 1.8 | 1.8 | 1.8 | 1.8 | 1.8 |
| **Natural and Artificial Vanilla Flavor** | 1.8 | 1.8 | 1.8 | 1.8 | 1.8 |
| **Sodium phosphate monobasic monohydrate** | 1.6 | 1.6 | 1.6 | 1.6 | 1.6 |
| **Potassium phosphate dibasic trihydrate** | 1.1 | 1.1 | 1.1 | 1.1 | 1.1 |
| **Art. Cotton candy flavor** | 1.0 | 1.0 | 1.0 | 1.0 | 1.0 |
| **Vitamin premix** | 1.0 | 1.0 | 1.0 | 1.0 | 1.0 |
| **Ascorbyl plamitate** | 0.2437 | 0.2437 | 0.2437 | 0.2437 | 0.2437 |
| **Ascorbic acid** | 0.2400 | 0.2400 | 0.2400 | 0.2400 | 0.2400 |
| **Tocopherol-2 food grade antioxidant** | 0.1161 | 0.1161 | 0.1161 | 0.1161 | 0.1161 |
| **Ferrous sulfate** | 0.0929 | 0.0929 | 0.0929 | 0.0929 | 0.0929 |
| **Vitamin premix** | 0.065 | 0.065 | 0.065 | 0.065 | 0.065 |
| **Zinc sulfate monohydrate** | 0.057 | 0.057 | 0.057 | 0.057 | 0.057 |
| **Manganese sulfate** | 0.0454 | 0.0454 | 0.0454 | 0.0454 | 0.0454 |
| **Mineral mix copper sulfate** | 0.0353 | 0.0353 | 0.0353 | 0.0353 | 0.0353 |
| **Beta carotene 30%** | 0.0056 | 0.0056 | 0.0056 | 0.0056 | 0.0056 |
| **Chromium chloride** | 0.0013 | 0.0013 | 0.0013 | 0.0013 | 0.0013 |
| **Sodium molybdate** | 0.0012 | 0.0012 | 0.0012 | 0.0012 | 0.0012 |
| **Potassium iodide** | 0.001 | 0.001 | 0.001 | 0.001 | 0.001 |
| **Sodium selenite** | 0.0004008 | 0.0004008 | 0.0004008 | 0.0004008 | 0.0004008 |
| **Citric acid** | As needed | As needed | As needed | As needed | As needed |
| **Potassium hydroxide** | As needed | As needed | As needed | As needed | As needed |
| **Magnesium sulfate dry** | As needed | As needed | As needed | As needed | As needed |
| **Ultra micronized tricalcium phosphate** | As needed | As needed | As needed | As needed | As needed |
| **Ascorbic acid** | As needed | As needed | As needed | As needed | As needed |
| **Water** | q.s. | q.s. | q.s. | q.s. | q.s. |

| **Ingredients** | **Amounts (kg) per 1000 kg** | | | | |
|---|---|---|---|---|---|
| | **Example 110** | **Example 111** | **Example 112** | **Example 113** | **Example 114** |
| **Maltrin** | 436.7 | 436.7 | 436.7 | 436.7 | 436.7 |
| **Sucrose** | 145.5 | 145.5 | 145.5 | 145.5 | 145.5 |
| **Calcium Caseinate** | 64.6 | 51.6 | 38.7 | 25.8 | 12.91 |
| **Pea Protein Hydrolysate** | 93.4 | 112.1 | 130.8 | 149.4 | 168.1 |
| **Conjugated Linoleic Acid** | 67.0 | 63.65 | 70.35 | 60.3 | 73.7 |
| **Isolated Soy Protein** | 28.8 | 23.1 | 17.3 | 11.5 | 5.77 |
| **FOS Powder** | 33.6 | 33.6 | 33.6 | 33.6 | 33.6 |
| **High oleic sunflower oil** | 33.1 | 33.1 | 33.1 | 33.1 | 33.1 |
| **Calcium β-hydroxy-β-methylbutarate** | 31.6 | 31.6 | 31.6 | 31.6 | 31.6 |
| **Canola Oil** | 28.3 | 28.3 | 28.3 | 28.3 | 28.3 |
| **Soy Oil** | 13.3 | 13.3 | 13.3 | 13,3 | 13.3 |
| **Potassium Citrate** | 10.3 | 10.3 | 10.3 | 10.3 | 10.3 |
| **Sodium Citrate** | 5.8 | 5.8 | 5.8 | 5.8 | 5.8 |
| **Potassium Chloride** | 5.2 | 5.2 | 5.2 | 5.2 | 5.2 |
| **Magnesium Chloride** | 4.7 | 4.7 | 4.7 | 4.7 | 4.7 |
| **Potassium hydroxide** | 3.1 | 3.1 | 3.1 | 3.1 | 3.1 |
| **Sodium phosphate dibasic dehydrate** | 3.0 | 3.0 | 3.0 | 3.0 | 3.0 |
| **Sodium chloride** | 2.5 | 2.5 | 2.5 | 2.5 | 2.5 |
| **Choline Chloride** | 1.8 | 1.8 | 1.8 | 1.8 | 1.8 |
| **Natural and Artificial Vanilla Flavor** | 1.8 | 1.8 | 1.8 | 1.8 | 1.8 |
| **Sodium phosphate monobasic monohydrate** | 1.6 | 1.6 | 1.6 | 1.6 | 1.6 |
| **Potassium phosphate dibasic trihydrate** | 1.1 | 1.1 | 1.1 | 1.1 | 1.1 |
| **Art. Cotton candy flavor** | 1.0 | 1.0 | 1.0 | 1.0 | 1.0 |
| **Vitamin premix** | 1.0 | 1.0 | 1.0 | 1.0 | 1.0 |
| **Ascorbyl plamitate** | 0.2437 | 0.2437 | 0.2437 | 0.2437 | 0.2437 |
| **Ascorbic acid** | 0.2400 | 0.2400 | 0.2400 | 0.2400 | 0.2400 |
| **Tocopherol-2 food grade antioxidant** | 0.1161 | 0.1161 | 0.1161 | 0.1161 | 0.1161 |
| **Ferrous sulfate** | 0.0929 | 0.0929 | 0.0929 | 0.0929 | 0.0929 |
| **Vitamin premix** | 0.065 | 0.065 | 0.065 | 0.065 | 0.065 |
| **Zinc sulfate monohydrate** | 0.057 | 0.057 | 0.057 | 0.057 | 0.057 |
| **Manganese sulfate** | 0.0454 | 0.0454 | 0.0454 | 0.0454 | 0.0454 |
| **Mineral mix copper sulfate** | 0.0353 | 0.0353 | 0.0353 | 0.0353 | 0.0353 |
| **Beta carotene 30%** | 0.0056 | 0.0056 | 0.0056 | 0.0056 | 0.0056 |
| **Chromium chloride** | 0.0013 | 0.0013 | 0.0013 | 0.0013 | 0.0013 |
| **Sodium molybdate** | 0.0012 | 0,0012 | 0.0012 | 0.0012 | 0.0012 |
| **Potassium iodide** | 0.001 | 0.001 | 0.001 | 0.001 | 0.001 |
| **Sodium selenite** | 0.0004008 | 0.0004008 | 0.0004008 | 0.0004008 | 0.0004008 |
| **Citric acid** | As needed | As needed | As needed | As needed | As needed |
| **Potassium hydroxide** | As needed | As needed | As needed | As needed | As needed |
| **Magnesium sulfate dry** | As needed | As needed | As needed | As needed | As needed |
| **Ultra micronized tricalcium phosphate** | As needed | As needed | As needed | As needed | As needed |
| **Ascorbic acid** | As needed | As needed | As needed | As needed | As needed |
| **Water** | q.s. | q.s. | q.s. | q.s. | q.s. |

The invention is also illustrated by the following clauses:
1. A nutritional product comprising a pea protein hydrolysate having less than 15,000 µg of immunologically active pea antigen per 1 gm of protein.
2. The nutritional product of clause 1 wherein the pea protein hydrolysate has less than 10,000 µg of immunologically active pea antigen per 1 gm of protein.
3. The nutritional product of clause 1 wherein the pea protein hydrolysate further has an adjusted degree of hydrolysis of between about 7.0% and less than 19.0%.
4. The nutritional product of clause 1 wherein the product is a hypoallergenic product selected from the group consisting of a preterm infant formula, a human milk fortifier, and an infant formula.
5. The nutritional product of clause 1 further comprising a fat and a carbohydrate.
6. The nutritional product of clause 5 comprising from about 0.5% to about 30% by weight of protein.
7. The nutritional product of clause 6 comprising from about 1% to about 30% by weight of fat.
8. The nutritional product of clause 6 comprising from about 5% to about 40% by weight of carbohydrate.
9. A method of making a nutritional product having reduced immunological reactivity comprising combining a protein and a carbohydrate, wherein the protein comprises a pea protein hydrolysate having less than 15,000 µg of immunologically active pea antigen per 1 gm of protein.
10. The method of clause 9 further comprising combining a fat with the protein and the carbohydrate.
11. A method of reducing the incidence of allergic reactions to food in individuals with food-allergic allergies, the method comprising administering to the individual a composition comprising a pea protein hydrolysate having an adjusted degree of hydrolysis of between about 7.0% and less than 19.0%.
12. The method of clause 11 wherein the pea protein hydrolysate comprises less than 5% proteins having a molecular weight of greater than 3 kDa.
13. A method of reducing the incidence of allergic reactions to food in infants with food-allergic allergies, the method comprising administering to the infant a composition comprising a pea protein hydrolysate having an adjusted degree of hydrolysis of between about 7.0% and less than 19.0%
14. The method of clause 13 wherein the pea protein hydrolysate comprises less than 5% proteins having a molecular weight of greater than 3 kDa.
15. A method of reducing the incidence of intolerance to food in individuals with food intolerance, the method comprising administering to the individual a composition comprising a pea protein hydrolysate having an adjusted degree of hydrolysis of between about 7.0% and less than 19.0%.

## Claims

1. A pea protein hydrolysate having an adjusted degree of hydrolysis of between about 7% and less than 19%.

2. A pea protein hydrolysate having less than 15,000 µg of immunologically active pea antigen per 1 gm of protein.

3. The pea protein hydrolysate of claim 2, wherein the pea protein hydrolysate has an adjusted degree of hydrolysis of between about 7% and less than 19%.

4. The pea protein hydrolysate of either claim 1 or 3, wherein the adjusted degree of hydrolysis is between about 8% and about 16%.

5. The pea protein hydrolysate of either claim 1 or 3, wherein the adjusted degree of hydrolysis is between about 8% and about 14%.

6. The pea protein hydrolysate of either claim 1 or 3, wherein the adjusted degree of hydrolysis is between about 8% and about 13.5%.

7. A pea protein hydrolysate for use as a nutritional source for individuals who are intolerant or allergic, or both to cow milk proteins or soy proteins, or both, wherein the pea protein hydrolysate has less than 15,000 µg of immunologically active pea antigen per 1 gm of protein and has an adjusted degree of hydrolysis of between about 7% and less than 19%.

8. Use of a pea protein hydrolysate for preparing a nutritional product for individuals who are intolerant or allergic, or both to cow milk proteins or soy proteins, or both, wherein the pea protein hydrolysate has less than 15,000 µg of immunologically active pea antigen per 1 gm of protein and has an adjusted degree of hydrolysis of between about 7% and less than 19%.

9. Pea protein hydrolysate having an adjusted degree of hydrolysis of between about 7% and less than 19% for use in a method of reducing the incidence of allergic reactions to food in individuals with a food-allergic allergy by administration of the pea protein hydrolysate to the individual.

10. Pea protein hydrolysate having an adjusted degree of hydrolysis of between about 7% and less than 19% for use in a method of reducing the incidence of intolerance to food in individuals with a food intolerance by administration of the pea protein hydrolysate to the individual.

11. Use of a pea protein hydrolysate according to claim 8 or the pea protein for use according to claim 7, 9 or 10, wherein the pea protein hydrolysate has an adjusted degree of hydrolysis of between about 8% and about 16%.

12. Use of a pea protein hydrolysate according to claim 8 or the pea protein for use according to claim 7,9 or 10, wherein the individual is an infant.
